(19) Europäisches Patentamt
European Patent Office
Office européen des brevets

(11) EP 3 551 679 B1

(12) **EUROPEAN PATENT SPECIFICATION**

(45) Date of publication and mention of the grant of the patent:
**24.05.2023 Bulletin 2023/21**

(21) Application number: **17807887.9**

(22) Date of filing: **04.12.2017**

(51) International Patent Classification (IPC):
***C08F 220/58*** *(2006.01)*

(52) Cooperative Patent Classification (CPC):
(C-Sets available)
**C08F 220/585** (Cont.)

(86) International application number:
**PCT/EP2017/081414**

(87) International publication number:
**WO 2018/108608 (21.06.2018 Gazette 2018/25)**

(54) **POLYMER COMPRISING CERTAIN LEVEL OF BIO-BASED CARBON**

POLYMER MIT EINEM BESTIMMTEN GRAD AN BIOBASIERTEM KOHLENSTOFF

POLYMÈRE COMPRENANT UN CERTAIN NIVEAU DE CARBONE BIOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.12.2016 EP 16203555**

(43) Date of publication of application:
**16.10.2019 Bulletin 2019/42**

(73) Proprietor: **Clariant International Ltd**
**4132 Muttenz (CH)**

(72) Inventors:
- **FISCHER, Dirk**
  **55278 Hahnheim (DE)**
- **KAYSER, Christoph**
  **55127 Mainz (DE)**
- **STARKULLA, Gundula**
  **55130 Mainz (DE)**

(74) Representative: **Clariant Produkte (Deutschland) GmbH**
**Patent Management**
**Industriepark Höchst, G 860**
**DE-65926 Frankfurt am Main (DE)**

(56) References cited:
**EP-A2- 0 750 899**
**EP-A2- 0 816 403**
**WO-A1-98/00094**
**WO-A1-2012/084977**
**WO-A1-2013/113938**
**US-A1- 2005 003 984**
**US-A1- 2006 019 835**
**US-A1- 2010 278 763**
**US-A1- 2011 110 878**
**US-A1- 2014 086 854**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

**(Cont. next page)**

(52) Cooperative Patent Classification (CPC): (Cont.)

C-Sets
C08F 220/585, C08F 220/06, C08F 220/14, C08F 222/103;
C08F 220/585, C08F 220/06, C08F 220/14, C08F 222/104;
C08F 220/585, C08F 220/06, C08F 220/14, C08F 222/1063;
C08F 220/585, C08F 220/06, C08F 220/54, C08F 222/103;
C08F 220/585, C08F 220/06, C08F 220/54, C08F 222/104;
C08F 220/585, C08F 220/06, C08F 220/54, C08F 222/1063

## Description

FIELD OF THE INVENTION

The present invention relates to a polymer having a certain bio-based carbon content obtained by polymerizing, in addition to processes and uses in cosmetic applications.

BACKGROUND OF THE INVENTION

Cleansing and caring for the skin, scalp, and hair is very important for general hygiene e.g. for removal of unwanted materials such as sebum, oils, dirt, makeup, or for moisturisation, colouring or protection. Many cosmetic products require a certain minimum viscosity in order to achieve ease of application to the substrate and/or retention on the substrate to be treated. Many cosmetic products comprise viscosity-increasing or rheology-influencing agents. These are often referred to as thickening agents, thickeners or gelling agents. Thickening agents used in cosmetics or personal hygiene products include viscous liquids such as polyethylene glycol, synthetic polymers such as polyacrylic acid and vegetable gums. In the 1990s, innovative thickeners based on 2-acrylamido-2-methyl-1-propanesulfonic acid (AMPS) and their salts were introduced into the market (EP0816403 and WO98/00094). In both homopolymer and copolymer form (e.g. Aristoflex® AVC from Clariant), such thickeners are superior in many respects to the corresponding polycarboxylates (Carbopols). US2014/0086854 discloses polymers based on sulfonic acids, amides and cross-linking agents.

Many materials employed for use as thickeners or rheology modifiers are traditionally derived from crude oil. Environmental, economic and sustainability questions are restricting the use of products derived from this limited resource: synthetic surfactants, for example, have been blamed for environmental incidents, particularly vis-à-vis aquatic problems in rivers and lakes. Therefore, there is a desire to identify more sustainable and biodegradable, yet gentle and effective materials. Indeed, consumers are very interested in "natural" products including products with a high percentage of "natural" compounds and/or compounds that are derived from renewable materials. Consumers perceive compounds derived from natural materials to be gentler and more environmentally friendly. Recent industrial developments in "bio-based" chemicals are summarised, for example, in de Jong et al, "Product developments in the bio-based chemicals arena", Biofuels, Bioprod. Bioref. 6:606-624 (2012).

Compounds derived from natural materials have various other benefits, including increased biodegradability and also more sustainable availability because they are not based on a limited resource. Compounds derived from plant-based resources are particularly useful since the source compound can simply be regrown. Consumers are also particularly comfortable with using compounds derived from well-known plants, especially those that are considered staple products.

Recently, classical monomers such as ethylene, acrylic acid or methyl methacrylate have been disclosed as being produced with renewable raw materials. US2014/0154758 (Arkema) discloses the preparation of methyl methacrylate wherein the method comprises the use of acetone cyanohydrin as a raw material, said acetone cyanohydrin being obtained by condensing cyanohydric acid in acetone, and the methyl methacrylate is prepared using a process involving the addition of methanol. Acetone and methanol can be sourced from renewable feedstock. DE 2655891 (DU PONT) discloses the oxidation from 1-propanol to acrylates. US 4,138,430 (DU PONT) discloses the ammoxidation of 1-propanol to form acrylonitrile.

Different synthetic routes for the synthesis of bio-based acrylonitrile are described by M. Olga Guerrero-Péreza and Miguel A. Bañares in Catalysis Today 239 (2015) 25-30. The process for the direct production of acrylonitrile from glycerol was described recently by M.O. Guerrero-Pérez, M.A. Bañares, ChemSusChem 1 (2008) 511 and by M.A. Bañares, M.O. Guerrero-Pérez, Appl. Catal. B (2013), as well as in US20100048850A1 (Arkema) and WO2009063120A1 (CONSEJO SUPERIOR DE INVESTIGACIONES CIENTÍFICAS).

Bio-based propylene can directly been used in the so-called SOHIO process to form acrylonitrile. US2904580 (STANDARD OIL CO) describes the ammoxidation of propylene according to the so-called SOHIO process.

WO2014086780 (Global Bioenergies) discloses a fermentation method for several olefins including propene and isobutene. As seen before propene can be used as a raw material for the ammoxidation to acrylonitrile. Isobutene is an important raw material for polyisobutene rubbers and other downstream products such as tert.-butanol, iso-octanol, branched alkanes or branched alcohols. WO2016/042011 (Global Bioenergies) describes an enzymatic method for the production of isobutene from 3-methylcrotonyl-CoA. WO2014/004616 (Gevo Inc) discloses the synthesis of isobutanol by recombinant yeast microorganisms. The catalytic dehydration leads to isobutene.

WO2015/034948 (MYRIANT CORP) describes the synthesis of bio-based acrylic acid by dehydration of 1.3-propandiol and subsequent oxidation of the allylic alcohol.

WO2013/113938 discloses polymers obtained from 2-methylidenebutanedioate diester monomers. Preferably, the polymers and/or monomers are obtained from bio-renewable sources.

Nevertheless, the availability of more renewable polymers suitable for use as thickening agents is highly limited.

Furthermore, there is a need for thickening agents that are not only more renewable, but also provide excellent performance. There is a need, therefore, for providing polymers that can provide the excellent performance of modern polymers yet from more sustainable sources.

## SUMMARY OF THE INVENTION

In a first aspect, the present invention relates to a polymer comprising:

(a) from 9.49 mol-% to 98 mol-%, preferably from 27.5 mol-% to 97.4 mol-% repeating units (a) resulting from the incorporation of acryloyldimethyltaurate wherein at least 10 wt.-%, preferably at least 20 wt.-% of the repeating units resulting from the incorporation of acryloyldimethyltaurate comprise from 28 wt.-% to 100 wt.-% bio-based carbon content, relative to the total mass of carbon in the repeating unit (a) resulting from the incorporation of acryloyldimethyltaurate, measured according to standard ASTM D6866-12, Method B;

wherein the acryloyldimethyltaurate is prepared from acrylonitrile, isobutene and a mixture of sulfuric acid and fuming sulfuric acid comprising sulfur trioxide, wherein at least one of acrylonitrile and isobutene is of bio-based origin, and
wherein the sulfonic acid groups of the repeating units resulting from the incorporation of acryloyldimethyltaurate comprise counterions $Q^+$,
wherein $Q^+$ is $H^+$, $NH_4^+$, organic ammonium ions $[NHR^5R^6R^7]^+$ wherein $R^5$, $R^6$, and $R^7$ independently of one another may be hydrogen, a linear or branched alkyl group having 1 to 22 carbon atoms, a linear or branched, singularly or multiply unsaturated alkenyl group having 2 to 22 carbon atoms, a $C_6$-$C_{22}$ alkylamidopropyl group, a linear mono-hydroxyalkyl group having 2 to carbon atoms or a linear or branched dihydroxyalkyl group having 3 to carbon atoms, and where at least one of the radicals $R^5$, $R^6$, and $R^7$ is not hydrogen, or $Q^+$ is $Li^+$, $Na^+$, $K^+$, ½ $Ca^{++}$, ½ $Mg^{++}$, ½ $Zn^{++}$, 1/3 $Al^{+++}$, or combinations thereof;

(b) from 0.01 mol-% to 5 mol-%, preferably from 0.01 mol-% to 4 mol-% crosslinking or branching units, wherein the crosslinking or branching units result from the incorporation of a monomer comprising at least two olefinically unsaturated double bonds;

(c) from 0.01 mol-% to 88.52 mol-%, preferably from 0.05 mol-% to 72.4 mol-% of repeating neutral structural units, preferably wherein at least 10%, preferably at least 20 wt.-% of the repeating neutral structural units comprise from 0 wt.-% to 100 wt.-% bio-based carbon content, relative to the total mass of carbon in the repeating neutral structural unit, measured according to standard ASTM D6866-12, Method B;

(d) from 1.98 mol-% to 20 mol-%, preferably from 2.5 mol-% to 18 mol-% of repeating anionic structural units, wherein the repeating anionic structural units result from the incorporation of a monomer comprising at least one carboxylate anion, and wherein the repeating anionic structural units are different from units (a), preferably wherein at least 10 wt.-%, preferably at least 20 wt.-% of the repeating anionic structural units comprise from 0 wt.-% to 100 wt.-% bio-based carbon content, relative to the total mass of carbon in the repeating anionic structural unit, measured according to standard ASTM D6866-12, Method B.

Other aspects relate to compositions, methods, uses, and processes related to the polymer disclosed in the first aspect.

## DETAILED DESCRIPTION OF THE INVENTION

### Definitions and general

In this document, including in all embodiments of all aspects of the present invention, the following definitions apply unless specifically stated otherwise. All percentages are by weight (w/w) of the total composition. "wt.-%" means percentage by weight; "vol.-%" means percentage by volume; "mol.-%" means percentage by mole. All ratios are weight ratios. References to 'parts' e.g. a mixture of 1 part X and 3 parts Y, is a ratio by weight. "QS" or "QSP" means sufficient quantity for 100 % or for 100 g. +/- indicates the standard deviation. All ranges are inclusive and combinable. The number of significant digits conveys neither a limitation on the indicated amounts nor on the accuracy of the measurements. All measurements are understood to be made at 23 °C and at ambient conditions, where "ambient conditions" means at 1 atmosphere (atm) of pressure and at 50 % relative humidity. "Relative humidity" refers to the ratio (stated as a percent) of the moisture content of air compared to the saturated moisture level at the same temperature and pressure. Relative

humidity can be measured with a hygrometer, in particular with a probe hygrometer from VWR® International. Herein "min" means "minute" or "minutes". Herein "mol" means mole.

Herein "g" following a number means "gram" or "grams". "Ex." means "example". All amounts as they pertain to listed ingredients are based on the active level ('solids') and do not include carriers or by-products that may be included in commercially available materials. Herein, "comprising" means that other steps and other ingredients can be in addition. "Comprising" encompasses the terms "consisting of" and "consisting essentially of". The compositions, formulations, methods, uses, kits, and processes of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated. "In at least one embodiment" means that one or more embodiments, optionally all embodiments or a large subset of embodiments, of the present invention has/have the subsequently described feature. Where amount ranges are given, these are to be understood as being the total amount of said ingredient in the composition, or where more than one species fall within the scope of the ingredient definition, the total amount of all ingredients fitting that definition, in the composition. For example, if the composition comprises from 1 % to 5 % fatty alcohol, then a composition comprising 2 % stearyl alcohol and 1 % cetyl alcohol and no other fatty alcohol, would fall within this scope.

The following acronyms are used herein: ACDMT = acryloyldimethyltaurate; AM = acrylamide; AN = acrylonitrile; tBAM = tert.-butyl acrylamide; IBSA = isobutene sulfonic acid; IBDSA = 2-methylidene-1,3-propylenedisulfonic acid.

Unless otherwise stated, "viscosity" herein is measured at 20 °C viscosity in centipoise (cP) or mPa.s using a Brookfield viscometer model LV, RVT DV-II or LVT DV-II with 10 - 90 % torque at 20 rpm.

"Molecular weight" or "M.Wt." "Mw", "$M_w$" or "MW" and grammatical equivalents mean the weight average molecular weight, unless otherwise stated. Also relevant for the determination of the molecular weight distribution is the number average molecular weight "Mn", "$M_n$" and grammatical equivalents, and the polydispersity "D" or "PDI".

Number average molecular weight: Mn

The number average molecular weight is the statistical average molecular weight of all the polymer chains in the sample, and is defined by:

$$Mn = \frac{\sum NiMi}{\sum Ni}$$

where $M_i$ is the molecular weight of a chain and $N_i$ is the number of chains of that molecular weight. $M_n$ can be predicted by polymerisation mechanisms and is measured by methods that determine the number of molecules in a sample of a given weight; for example, colligative methods such as end-group assay. If $M_n$ is quoted for a molecular weight distribution, there are equal numbers of molecules on either side of $M_n$ in the distribution.

Weight average molecular weight: Mw

The weight average molecular weight is defined by:

$$MW = \frac{\sum NiMi^2}{\sum NiMi}$$

Compared to $M_n$, Mw takes into account the molecular weight of a chain in determining contributions to the molecular weight average. The more massive the chain, the more the chain contributes to Mw. Mw is determined by methods that are sensitive to the molecular size rather than just their number, such as light scattering techniques. If Mw is quoted for the molecular weight distribution, there is an equal weight of molecules on either side of Mw in the distribution.

The polydispersity index PDI is used as a measure of the broadness of a molecular weight distribution of a polymer, and is defined by:

$$PDI = \frac{MW}{Mn}$$

The larger the PDI, the broader the molecular weight. A monodisperse polymer where all the chain lengths are equal (such as a protein) has an $M_W/M_n=1$.

The weight average molecular weight can be measured by gel permeation chromatography (GPC), also referred to as size exclusion chromatography (SEC). The molecular weight of polymers and its measurement is described in the textbook "Principles of Polymerization" by Georg Odian, third edition, Wiley-Interscience, New York, in chapter 1-4, page 19 to 24, ISBN 0-471-61020-8. The process to determine the weight average molecular weight is described in detail in chapter 3 of Makromolekulare Chemie: Eine Einführung" by Bernd Tieke, Wiley-VCH, 2. vollständig überarbeitete und erweiterte Auflage (3. Nachdruck 2010) ISBN-13: 978-3-527-31379-2, page 259-261.

Determination of molecular weight and distribution of samples by

GPC is determined under the following conditions.
Column: PSS Suprema 30,000 Å 10 μm, 300 mm × 8 mm
Detector: RID
Oven temperature: 23 °C
Flow: 1 ml/min
Injection volume: 20 μl
Eluent: 0.07 mol/l disodium hydrogen phosphate in water
Calibration method: Conventional poly(styrene sulfonate) sodium salt calibration Sample preparation: Weigh approx. 10 mg sample in 10 ml 0.07 mol/l disodium hydrogen phosphate in water and shake for 15 min.

"Water-soluble" refers to any material that is sufficiently soluble in water to form a clear solution to the naked eye at a concentration of 0.1 % by weight of the material in water at 25 °C. The term "water-insoluble" refers to any material that is not "water-soluble".

"Substantially free from" or "substantially free of" means less than 1 %, or less than 0.8 %, or less than 0.5 %, or less than 0.3 %, or 0 %, by total weight of the composition or formulation.

"Monomer" means a discrete, non-polymerised chemical moiety capable of undergoing polymerisation in the presence of an initiator or any suitable reaction that creates a macromolecule e.g. such as radical polymerisation, polycondensation, polyaddition, anionic or cationic polymerization, ring opening polymerisation or coordination insertion polymerisation. "Unit" means a monomer that has already been polymerised i.e. is part of a polymer.

"Polymer" means a chemical formed from the polymerisation of two or more monomers. The term "polymer" shall include all materials made by the polymerisation of monomers as well as natural polymers. Polymers made from only one type of monomer are called homopolymers. Herein, a polymer comprises at least two monomers. Polymers made from two or more different types of monomers are called copolymers. The distribution of the different monomers can be random, alternating or block-wise (i.e. block copolymer). The term "polymer" used herein includes any type of polymer including homopolymers and copolymers.

"Fuming sulfuric acid" herein means a solution of sulfur trioxide in sulfuric acid. Fuming sulfuric acid is also known as oleum and is identified by the CAS number 8014-95-7, and can be described by the formula $H_2SO_4.xSO_3$ where *x* is the molar free sulfur trioxide content.

The "biobased content" is reported in ASTM D6866-12, Method B (see section 3.3.9 of ASTM D6866-12). "Biobased carbon content", "biobased content", "biogenic carbon content", "bio-based content", "biomass-derived carbon" herein refer to the same thing and are all measured in wt.-%. Herein, the term 'bio-based carbon content' is used. ASTM D6866-12, Method B lab results report the percentage of bio-based carbon content relative to total carbon, and not to total mass of the sample or molecular weight. A comment on bio-based carbon content calculation: Presently ASTM D6866-12, Method B (see section 9 of ASTM D6866-12) requires the percent modern carbon value (pMC) reported to be multiplied by a correction factor of 0.95 to account for excess carbon-14 in the atmosphere due to nuclear weapons testing. However, a revision is pending for ASTM D6866-12, Method B to update the correction factor to 0.98 due to ongoing decrease in excess atmospheric $^{14}CO_2$. For the purposes of accuracy, the new correction factor of 0.98 is often reported in the field e.g. by suppliers. Generally, results below ~20 % bio-based carbon will not be affected. However, results close to 100% will be ~2-3 % bio-based carbon higher using the 0.98 factor vs 0.95. Results between ~20-90 % will increase by 0-3 %. Hence the term "bio-based carbon content" as used herein is defined by the equation:

$$\text{Bio-based carbon content} = \text{pMC} * 0.95\ (\%)$$

A review on measurement methods of bio-based carbon content for biomass-based chemicals and plastics is given by Massao Kunioka in Radioisotopes, 62, 901-925 (2013).

"Hair" means mammalian keratin fibres including scalp hair, facial hair and body hair. It includes such hair still being attached to a living subject and also hair that has been removed therefrom such as hair swatches and hair on a

doll/mannequin. In at least one embodiment, "hair" means human hair. "Hair shaft" or "hair fibre" means an individual hair strand and may be used interchangeably with the term "hair."

"Cosmetically acceptable" means that the compositions, formulations or components described are suitable for use in contact with human keratinous tissue without undue toxicity, incompatibility, instability, allergic response. All compositions and formulations described herein which have the purpose of being directly applied to keratinous tissue are limited to those being cosmetically acceptable.

"Derivatives" includes but is not limited to, amide, ether, ester, amino, carboxyl, acetyl, acid, salts and/or alcohol derivatives of a given compound. In at least one embodiment, "derivatives thereof" means the amide, ether, ester, amino, carboxyl, acetyl, acid, salt and alcohol derivatives.

Explanation of and benefits provided by the invention

Surprisingly, it has now been found that it is possible to synthesise good quality bio-based ACDMT (see Formula (3) below) at acceptable yields.

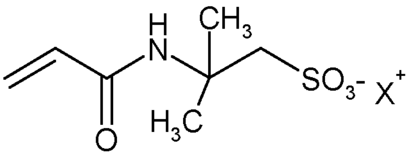

(3)

Indeed, when considering genetically-engineered microbes for use in creating bio-based ACDMT, currently no such microbes are commercially available. ACDMT itself is not similar to any other products that typical microbes would produce naturally. Furthermore, there are few natural microbial pathways capable of converting sulfonic acid groups. Therefore, the person skilled in the art naturally has a bias in his mind that it would be difficult to produce bio-based ACDMT in view of its more synthetic-type chemical moieties. The person skilled in the art, may however, consider that the reaction of acrylic acid with taurine, as bio-based materials could form the corresponding acryl-amido taurate compound, which is a similar structure as compared to ACDMT. However, the reactants would preferentiality react to form a Michael adduct, rather than an acryl-amido taurate compound. Hence, it would be known to the person skilled in the art that synthesising bio-based ACDMT is no trivial matter.

Bianca et al (Appl Microbiol Biotechnol (2012) 93:1377-1387) states that a high level of impurities are produced when bio-based isobutene is synthesised (2/3 carbon dioxide). WO2014086780A2 on pages 5 and 6 mentions various by-products and impurities that may result from the bio-based isobutene is synthesised. Indeed, on page 14 of WO2014086780A2 it states "The fermentation off-gas (i.e. a gas stream originating from the fermenter) typically comprises the hydrocarbon as the desired product and the intermediate together with additional gaseous components. Generally, the total content of the desired product, such as isobutene, and the intermediate, such as acetone, in the fermentation off-gas is in a range of 3 to 30 vol. %, preferably 3 to 20 vol. %". In other words, it is known in the art that a very low yield results when known bio-based isobutene synthesis processes are employed, as well as that a significant level of by-products is produced. Indeed, normally at least 98%, typically at least 99.5% purity of isobutene is used in conventional synthesis techniques. Surprisingly, it is possible to produce bio-based ACDMT despite using bio-based components that are typically impure in view of the microbes that produce the bio-based component creating by-products as a result of their natural enzymatic action. European patent application 16175218.3 filed on 20th June 2016 in the name of Clariant International Ltd discloses the synthesis of bio-based acryloyldimethyltaurate, which can be used as a monomer for the polymer according to the present invention.

Furthermore it has surprisingly been found that polymers containing such novel bio-based components can be synthesised. Such polymers may be, for example, crosslinked copolymers.

The present invention relates *inter alia* to polymers containing units derived from bio-based acryloyldimethyltaurate (ACDMT) and similar compounds. The preparation method of ACDMT typically comprises the use of acrylonitrile, isobutene and a mixture of sulfuric acid and fuming sulfuric acid comprising sulfur trioxide. Preferably, at least one of the raw materials, acrylonitrile or isobutene, are of bio-based origin. The bio-based ACDMT is suitable to make polymers comprising a bio-based carbon content stemming from its bio-based ACDMT share.

ACDMT (see Formula [3]) consists of seven carbon atoms. Preferably a minimum of three, preferably four and most preferred all seven carbon atoms of the ACDMT molecule can become renewable, bio-based carbon atoms. In this way, a high proportion of bio-based and/or biodegradable (polymer) products made from the bio-based monomer ACDMT are recyclable and part of the natural carbon cycle. If these kinds of products are incinerated or biodegraded, the quantity of carbon dioxide that is emitted corresponds to the quantity fixed by photosynthesis during biomass growth.

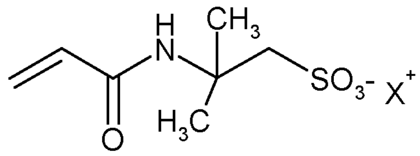

(3)

To date several high performance water soluble or water swellable polymers such as Fluid Loss Additives for the construction and (oil and gas) well construction industry as well as rheology modifiers, comprise ACDMT. Independent from the excellent performance in their applications, such polymers have so-far all been made from petrochemical based, fossil hydrocarbon based ACDMT. The present invention provides new polymers comprising units from bio-based ACDMT or similar compounds, thus giving access to new bio-based polymers having the excellent performance benefits that such conventionally synthetic polymers are known for.

The details of the invention and its aspects are provided hereinafter.

FIRST ASPECT

In a first aspect, the present invention relates to a polymer comprising:

(a) from 9.49 mol-% to 98 mol-%, preferably from 27.5 mol-% to 97.4 mol-% repeating units (a) resulting from the incorporation of acryloyldimethyltaurate wherein at least 10 wt.-%, preferably at least 20 wt.-% of the repeating units resulting from the incorporation of acryloyldimethyltaurate comprise from 28 wt.-% to 100 wt.-% bio-based carbon content, relative to the total mass of carbon in the repeating unit (a) resulting from the incorporation of acryloyldimethyltaurate, measured according to standard ASTM D6866-12, Method B;

wherein the acryloyldimethyltaurate is prepared from acrylonitrile, isobutene and a mixture of sulfuric acid and fuming sulfuric acid comprising sulfur trioxide, wherein at least one of acrylonitrile and isobutene is of bio-based origin, and
wherein the sulfonic acid groups of the repeating units resulting from the incorporation of acryloyldimethyltaurate comprise counterions $Q^+$,
wherein $Q^+$ is $H^+$, $NH_4^+$, organic ammonium ions $[NHR^5R^6R^7]^+$ wherein $R^5$, $R^6$, and $R^7$ independently of one another may be hydrogen, a linear or branched alkyl group having 1 to 22 carbon atoms, a linear or branched, singularly or multiply unsaturated alkenyl group having 2 to 22 carbon atoms, a $C_6$-$C_{22}$ alkylamidopropyl group, a linear mono-hydroxyalkyl group having 2 to carbon atoms or a linear or branched dihydroxyalkyl group having 3 to carbon atoms, and where at least one of the radicals $R^5$, $R^6$, and $R^7$ is not hydrogen, or $Q^+$ is $Li^+$, $Na^+$, $K^+$, ½ $Ca^{++}$, ½ $Mg^{++}$, ½ $Zn^{++}$, 1/3 $Al^{+++}$, or combinations thereof;

(b) from 0.01 mol-% to 5 mol-%, preferably from 0.01 mol-% to 4 mol-% crosslinking or branching units, wherein the crosslinking or branching units result from the incorporation of a monomer comprising at least two olefinically unsaturated double bonds;

(c) from 0.01 mol-% to 88.52 mol-%, preferably from 0.05 mol-% to 72.4 mol-% of repeating neutral structural units, preferably wherein at least 10 wt.-%, preferably at least 20 wt.-% of the repeating neutral structural units comprise from 0 wt.-% to 100 wt.-% bio-based carbon content, relative to the total mass of carbon in the repeating neutral structural unit, measured according to standard ASTM D6866-12, Method B;

(d) from 1.98 mol-% to 20 mol-%, preferably from 2.5 mol-% to 18 mol-% of repeating anionic structural units, wherein the repeating anionic structural units result from the incorporation of a monomer comprising at least one carboxylate anion, and wherein the repeating anionic structural units are different from units (a), preferably wherein at least 10 wt.-%, preferably at least 20 wt.-% of the repeating anionic structural units comprise from 0 wt.-% to 100 wt.-% bio-based carbon content, relative to the total mass of carbon in the repeating anionic structural unit, measured according to standard ASTM D6866-12, Method B.

In at least one embodiment, the polymer comprises from 37 mol-% to 96.4 mol-%, preferably from 43 mol-% to 95.3 mol-% units (a), from 0.1 mol-% to 3 mol-%, preferably from 0.2 mol-% to 2 mol-% units (b), from 0.1 mol-% to 59.3 mol-%, preferably from 0.5 mol-% to 52.8 mol-% units (c), and from 3.5 mol-% to 16 mol-%, preferably from 4 mol-% to 14 mol-% units (d). In at least one embodiment, the polymer comprises units (a), (b), (c) and (d) such that the sum thereof is at least 99 mol-%, by total weight of the polymer.

In at least one embodiment, the polymer comprises from 70 mol-% to 94.5 mol-%, units (a), from 0.35 mol-% to 1.5 mol-%, units (b), from 0.65 mol-% to 25.65 mol-% units (c), and from 4.5 mol-% to 12 mol-% units (d). In at least one embodiment, the polymer comprises units (a), (b), (c) and (d) such that the sum thereof is at least 99 mol-%, by total weight of the polymer.

In at least one embodiment, the polymer consists of units (a), units (b), units (c) and units (d).

In at least one embodiment, the polymer has a weight average molecular weight of at least 700 g/mol, preferably from 700 g/mol to 10 million g/mol.

Units (a)

The polymer comprises at least one repeating unit (a) resulting from the incorporation of acryloyldimethyltaurate wherein the sulfonic acid groups of the repeating units resulting from the incorporation of acryloyldimethyltaurate comprise counterions $Q^+$, wherein $Q^+$ is $H^+$, $NH_4^+$, $Li^+$, $Na^+$, $K^+$, ½ $Ca^{++}$, ½ $Mg^{++}$, ½ $Zn^{++}$, 1/3 $Al^{+++}$, or combinations thereof, preferably wherein $Q^+$ is $Na^+$or $NH_4^+$. $NH_4^+$ is preferred because it is more soluble the favored solvent used in the polymer synthesis. $Na^+$ is preferred because of reduced likelihood of unpreferred gases being produced during synthesis and also due to economic advantages.

In at least one embodiment, $Q^+$ is $NH_4^+$. In at least one embodiment, $Q^+$ is selected from the group monoalkylammonium, dialkylammonium, trialkylammonium and/or tetraalkylammonium salts, in which the alkyl substituents of the amines may independently of one another be ($C_1$ to $C_{22}$)-alkyl radicals or ($C_2$ to $C_{10}$)-hydroxyalkyl radicals.

In at least one embodiment, the polymer comprises at least one repeating unit (a) resulting from the incorporation of acryloyldimethyltaurate. In at least one embodiment, the polymer comprises two or more different repeating units (a) resulting from the incorporation of acryloyldimethyltaurate, such as repeating units resulting from the incorporation of acryloyldimethyltaurate having different $Q^+$ counterions.

In at least one embodiment, the repeating units (a) resulting from the incorporation of acryloyldimethyltaurate have a degree of neutralisation of between 0 mol-% and 100 mol-%. In at least one embodiment, the repeating units resulting from the incorporation of acryloyldimethyltaurate have a degree of neutralisation of from 50.0 to 100 mol-%, preferably from 80 mol-% to 100 mol-%, more preferably from 90.0 to 100 mol-%, even more preferably from 95.0 to 100 mol-%. Particular preference being given to a degree of neutralisation of more than 80 mol-%, more preferably more than 90 mol-%, even more preferably more than 95 mol-%. The degree of neutralisation is important in view of the molecular weight of the polymer and the yield of polymer produced.

At least 10 wt.-%, preferably at least 20 wt.-% of the repeating units (a) resulting from the incorporation of acryloyldimethyltaurate comprise from 28 wt.-% to 100 wt.-% bio-based carbon content, relative to the total mass of carbon in the repeating unit (a) resulting from the incorporation of acryloyldimethyltaurate, measured according to standard ASTM D6866-12, Method B. In at least one embodiment, at least 25 wt.-%, or at least 30 wt.-%, or at least 35 wt.-% , or at least 40 wt.-% , or at least 45 wt.-% , or at least 50 wt.-% , or at least 55 wt.-% , or at least 60 wt.-% , or at least 65 wt.-% , or at least 70 wt.-% , or at least 75 wt.-% , or at least 80 wt.-% , or at least 85 wt.-% , or at least 90 wt.-% , or at least 95 wt.-% , or at least 96 wt.-% , or at least 97 wt.-% , or at least 98 wt.-% , or at least 99 wt.-% , or at least 99.5 wt.-% of the repeating units resulting from the incorporation of acryloyldimethyltaurate comprise from 28 wt.-% to 100 wt.-% bio-based carbon content, relative to the total mass of carbon in the repeating unit (a) resulting from the incorporation of acryloyldimethyltaurate, measured according to standard ASTM D6866-12, Method B.

In at least one embodiment, the repeating units resulting from the incorporation of acryloyldimethyltaurate comprise from 28 wt.-% to 100 wt.-% bio-based carbon content, relative to the total mass of carbon in the repeating unit resulting from the incorporation of acryloyldimethyltaurate, measured according to standard ASTM D6866-12, Method B. In at least one embodiment, the repeating unit (a) comprises from 35 wt.-%, preferably from 40 wt.-%, more preferably from 54 wt.-%, even more preferably from 57 wt.-% to 100 wt.-%, most preferably 100 wt.-%, bio-based carbon content, relative to the total mass of carbon in the repeating unit resulting from the incorporation of acryloyldimethyltaurate, measured according to standard ASTM D6866-12, Method B.

The repeating units (a) result from the incorporation of acryloyldimethyltaurate.

In at least one embodiment, the polymer comprises from 55 mol-% to 98 mol-% of repeating units resulting from the incorporation of acryloyldimethyltaurate wherein at least 30 wt.-%, preferably at least 50 wt.-%, more preferably at least 70 wt.-% of the repeating units (a) resulting from the incorporation of acryloyldimethyltaurate comprise from 28 wt.-% to 100 wt.-% bio-based carbon content, relative to the total mass of carbon in the repeating unit, measured according to standard ASTM D6866-12, Method B;

Preferably the repeating units resulting from the incorporation of acryloyldimethyltaurate are incorporated by the polymerization of a compound according Formula (3), wherein X is a proton. More preferably the compound according to Formula (3) is ACDMT.

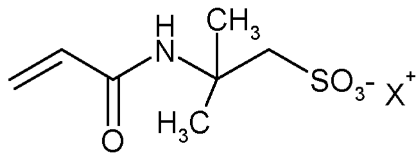

(3)

In at least one embodiment, the ACDMT comprises from 35 wt.-%, preferably from 40 wt.-%, more preferably from 54 wt.-%, even more preferably from 57 wt.-% to 100 wt.-%, most preferably 100 wt.-%, bio-based carbon content, relative to the total mass of carbon in ACDMT, measured according to standard ASTM D6866-12, Method B.

The bio-based carbon content, relative to the total mass of carbon in repeating units (a) resulting from the incorporation of acryloyldimethyltaurate, is measured according to standard ASTM D6866-12, Method B. More details on the analytical procedure for determination of bio-based carbon content: the provided sample material does not undergo any pre-treatment procedure and is converted to graphite as is using the following procedure:

Depending on the estimated amount of carbon content, typically a few milligrams of sample material is combusted in an elemental analyzer (EA). The resulting gas mixture is cleaned and $CO_2$ is automatically separated by the EA using the purge and trap technology. The remaining $CO_2$ is transferred into a custom-made graphitization system, converted into carbon (graphite) catalytically using $H_2$ and an iron-powder catalyst. The carbon-14 determination of the graphite is performed at the Klaus-Tschira-Archaeometrie-Center using an accelerator mass-spectrometer (AMS) of the type MICADAS (developed at the ETH Zurich, Switzerland).

Units (b)

The polymer comprises crosslinking or branching units (b), wherein the crosslinking or branching units result from the incorporation of a monomer comprising at least two olefinically unsaturated double bonds. The polymer comprises from 0.01 mol-% to 5 mol-%, preferably 0.01 mol-% to 4 mol-%, more preferably from 0.01 mol-% to 2 mol-% of crosslinking or branching units.

In at least one embodiment, the crosslinking or branching units comprise least one oxygen, nitrogen, and sulfur or phosphorus atom. In at least one embodiment, the crosslinking or branching units result from monomers having a molecular weight of less than 500 g/mol. In at least one embodiment, the units (b) are bifunctional or trifunctional crosslinking agents.

In at least one embodiment, the polymer comprises two or more different crosslinking or branching units.

In at least one embodiment, the crosslinking or branching units result from the incorporation of a monomer according to Formula (2):

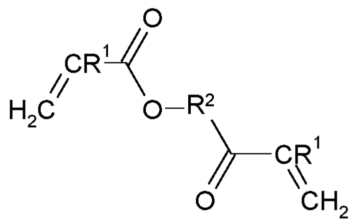

(2)

wherein

$R^1$ is independently selected from H, methyl or ethyl; and

$R^2$ is a linear or branched alkyl group having 1 to 6 carbon atoms, or is a linear or branched, mono- or polyunsaturated alkylene group having 2 to 6 carbon atoms, $-(CH_2-CH_2-O)_n-$

n is a integer from 1 to 100.

A monomer according to Formula (2) has the advantage that the polymer can be predicted as more brush-like in structure. However, brush-like polymers show different properties versus linear ones. For example, depending on different comonomer units the solubility could in- or decreased.

In at least one embodiment, the crosslinking or branching units result from the incorporation of a monomer according to Formula (4)

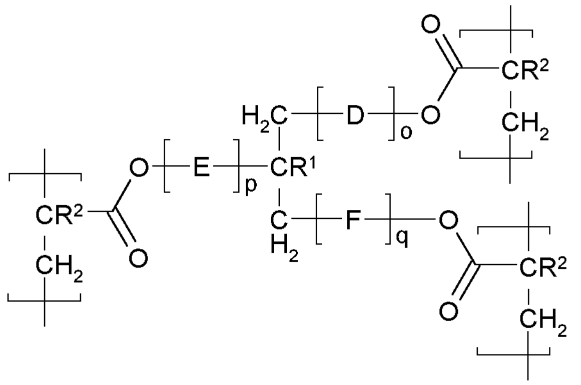

(4)

wherein

| | |
|---|---|
| $R^1$ | is independently selected from H, methyl or ethyl; and |
| $R^2$ | is a linear or branched alkyl group having 1 to 6 carbon atoms, or is a linear or branched, mono- or polyunsaturated alkylene group having 2 to 6 carbon atoms; |
| D, E, and F | are independently methyleneoxy($-CH_2O$), ethyleneoxy($-CH_2-CH_2-O-$), propyleneoxy($-CH(CH_3)-CH_2-O-$), a linear or branched alkylene group having 1 to 6 carbon atoms, a linear or branched, singularly or multiply unsaturated alkenylene group having 2 to 6 carbon atoms, a linear mono-hydroxyalkylene group having 2 to 6 carbon atoms or a linear or branched dihydroxyalkylene group having 3 to 6 carbon atoms; and |
| o, p, and q | each independently are an integer from 1 to 50. |

A monomer according to Formula (4) has the advantage that a polymer can be predicted as being highly branched.

In at least one embodiment, the crosslinking or branching units (b) result from the incorporation of a monomer selected from the group consisting of methylenebisacrylamide; methylenebismethacrylamide; esters of unsaturated monocarboxylic and polycarboxylic acids with polyols, preferably di-acrylates and tri-acrylates and -methacrylates (e.g. glycerol propoxylate triacrylate [GPTA]), more preferably butanediol and ethylene glycol diacrylate and poly ethylene glycol diacrylate and -methacrylate, trimethylolpropane triacrylate (TMPTA) and trimethylolpropane trimethacrylate (TMPTMA); allyl compounds, preferably allyl (meth)acrylate, triallyl cyanurate, diallyl maleate, polyallyl esters, tetraallyloxyethane, triallylamine, tetraallylethylenediamine; allyl esters of phosphoric acid; and/or vinylphosphonic acid derivatives. The choice of crosslinking or branching units is important in view of the flexibility of the crosslinks between the main chains of the polymer which affects the final performance of the polymer.

In at least one embodiment, the crosslinking or branching units (b) result from the incorporation of a crosslinker selected from the group consisting of trimethylolpropane triacrylate (TMPTA) and/or glycerol propoxylate triacrylate (GPTA). Particularly preferred as crosslinkers for the polymers of the invention are glycerol propoxylate triacrylate (GPTA), trimethylolpropane triacrylate (TMPTA), pentaerythritol diacrylate mono stearate (PEAS), hexanediol diacrylate (HDDA), poly ethylene glycol diacrylate (PEG-DA) and hexanediol dimethacrylate (HDDMA). Especially preferred is glycerol propoxylate triacrylate (GPTA) and trimethylolpropane triacrylate (TMPTA).

Units (c)

In at least one embodiment, the polymer at least one repeating neutral structural unit (c).

In at least one embodiment, the polymer comprises (c) from 0.99 mol-% to 59.99 mol-%, preferably from 1.99 mol-% to 44.99 mol-% of repeating neutral structural units (c); wherein the repeating neutral units comprise up to 100 wt.-% bio-based carbon content, relative to the total mass of carbon in the repeating unit, measured according to standard ASTM D6866-12, Method B.

In at least one embodiment, the polymer comprises at least one repeating neutral structural unit selected from the group consisting of N-vinylformamide, N-vinylacetamide, N-methyl-N-vinylformamide, N-methyl-N-vinylacetamide, N-vinyl-2-pyrrolidone, N-vinylcaprolactam, vinylacetate, N,N-dimethylacrylamide, N-isopropylacrylamide, acrylamide, methylacrylate, behenylpolyethoxy-(25)-methacrylate, laurylpoly-ethoxy-(7)-methacrylate, cetylpolyethoxy-(10)-methacrylate, stearylpoly-ethoxy-(8)-methacrylate, methoxypoly-ethoxy-(12)-methacrylate, and combinations thereof.

Units (d)

In at least one embodiment, the polymer comprises at least one repeating anionic structural unit (d).

In at least one embodiment, the polymer comprises from 1.98 mol-% to 20 mol-%, preferably from 2.5 mol-% to 18 mol-% of repeating anionic structural units, wherein the repeating anionic structural units result from the incorporation of a monomer comprising at least one carboxylate anion, and wherein the repeating anionic structural units (d) are different from units (a) and wherein the repeating anionic structural units comprises up to 100 wt.-% bio-based carbon content, relative to the total mass of carbon in the repeating unit, measured according to standard ASTM D6866-12, Method B.

In at least one embodiment, the repeating anionic structural unit results from the incorporation of monomers according to formula (A):

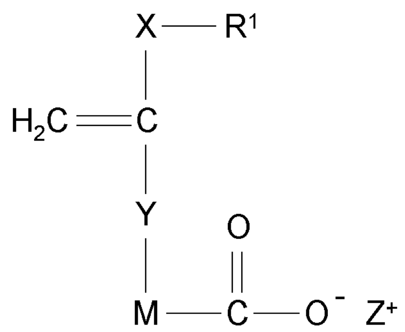

(A)

wherein

| | |
|---|---|
| $R^1$ and $R^3$ | are H, methyl or ethyl, or $C(O)O^-Z^+$; |
| X, Y | are selected from a covalent bond, O, $CH_2$, C(O)O, OC(O), $C(O)NR^3$ or $NR^3C(O)$; |
| M | are selected from a covalent bond, $-[C(O)O-CH_2-CH_2]_n-$, a linear or branched alkylene group with 1 to 6 carbon atoms, a linear or branched, mono- or polyunsaturated alkenylene group with 2 to 6 carbon atoms, a linear mono-hydroxyalkylene group with 2 to 6 carbon atoms or a linear or branched di-hydroxyalkylene group with 3 to 6 carbon atoms; |
| n | is an integer from 1 to 5; and |
| $Z^+$ | is $H^+$, $NH_4^+$, an organic ammonium ion $[HNR^5R^6R^7]^+$ wherein $R^5$, $R^6$ and $R^7$ are independently hydrogen, a linear or branched alkyl group with 1 to 22 carbon atoms, a linear or branched, mono- or polyunsaturated alkenyl group with 2 to 22 carbon atoms, a $C_6$ to $C_{22}$ alkylamidopropyl group, a linear mono-hydroxyalkyl group with 2 to 10 carbon atoms or a linear or branched di-hydroxyalkyl group with 3 to 10 carbon atoms, and wherein at least one of $R^5$, $R^6$ and $R^7$ is not hydrogen, or $Z^+$ is $Li^+$, $Na^+$, $K^+$, ½ $Ca^{++}$, ½ $Mg^{++}$, ½ $Zn^{++}$, 1/3 $Al^{+++}$, or combinations thereof. In at least one embodiment, the $Z^+$ is $H^+$, $NH_4^+$, $Li^+$, $Na^+$, $K^+$, ½ $Ca^{++}$, ½ $Mg^{++}$, ½ $Zn^{++}$, or 1/3 $Al^{+++}$, preferably $H^+$, $NH_4^+$, $Li^+$, $Na^+$ or $K^+$. |

In at least one embodiment, the polymer comprises at least one repeating anionic structural unit selected from the group consisting of acrylic acid or acrylate methacrylic acid or methacrylate, itaconic acid or itaconate, carboxyethylacrylic acid or carboxyethylacrylate, carboxyethylacrylic acid oligomers or carboxyethylacrylate oligomers, 2-propylacrylic acid or 2-propylacrylate, 2-ethylacrylic acid or 2-ethylacrylate, and their respective alkali or alkaline earth metal salts.

In at least one embodiment, the polymer comprises at least one repeating anionic structural unit selected from the group consisting of acrylic acid or acrylate methacrylic acid or methacrylate, itaconic acid or itaconate, carboxyethylacrylic acid or carboxyethylacrylate, carboxyethylacrylic acid oligomers or carboxyethylacrylate oligomers, and their respective alkali or alkaline earth metal salts. These repeating anionic structural units are preferred because they can easily be synthesised from bio-based sources.

Optional units (e)

In at least one embodiment, the polymer comprises at least one optional unit. In at least one embodiment, the optional unit results from the incorporation of a monomer selected from the group consisting of unsaturated carboxylic acids and their anhydrides and salts, and also their esters with aliphatic, olefinic, cycloaliphatic, arylaliphatic or aromatic alcohols having a carbon number of from 1 to 22. In at least one embodiment, the optional unit (e) results from the

incorporation of at least one monomer selected from the group consisting of functionalised (meth)acrylic acid esters, acrylic or methacrylic acid amides, polyglycol acrylic or methacrylic acid esters, polyglycol acrylic or methacrylic acid amides, dipropyleneglycolacrylic or methacrylic acid esters, dipropylenglycolacrylic or methacrylic acid amides, ethoxylated fatty alcohol acrylates or -methacrylates, propoxyl ated fatty alcohol acrylates or linear or cyclic N-vinylamides or N-methylvinyl amides.

In at least one embodiment, the optional unit results from the incorporation of monomers according to formula (A):

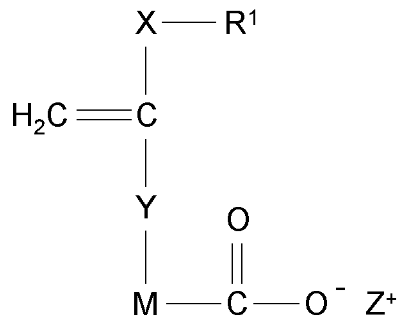

(A)

wherein:

| | |
|---|---|
| X, Y | are selected from a covalent bond, O, $CH_2$, C(O)O, OC(O), C(O)$NR^3$ or $NR^3$C(O); |
| $R^1$ and R | $^3$ are H, methyl or ethyl, or C(O)$O^-Z^+$; |
| M | is selected from a covalent bond, -[C(O)O-$CH_2$-$CH_2$]$_n$-, a linear or branched alkylene group with 1 to 6 carbon atoms, a linear or branched, mono- or polyunsaturated alkenylene group with 2 to 6 carbon atoms, a linear mono-hydroxyalkylene group with 2 to 6 carbon atoms or a linear or branched di-hydroxyalkylene group with 3 to 6 carbon atoms; |
| n | is an integer from 1 - 5, and |
| $Z^+$ | is $H^+$, $NH_4^+$, an organic ammonium ion $[HNR^5R^6R^7]^+$ wherein $R^5$, $R^6$ and $R^7$ are independently hydrogen, a linear or branched alkyl group with 1 to 22 carbon atoms, a linear or branched, mono- or polyunsaturated alkenyl group with 2 to 22 carbon atoms, a $C_6$ to $C_{22}$ alkylamidopropyl group, a linear mono-hydroxyalkyl group with 2 to 10 carbon atoms or a linear or branched di-hydroxyalkyl group with 3 to 10 carbon atoms, and wherein at least one of $R^5$, $R^6$ and $R^7$ is not hydrogen, or $Z^+$ is $Li^+$, $Na^+$, $K^+$, ½ $Ca^{++}$, ½ $Mg^{++}$, ½ $Zn^{++}$, 1/3 $Al^{+++}$, or combinations thereof. In at least one embodiment, the $Z^+$ is $H^+$, $NH_4^+$, $Li^+$, $Na^+$, $K^+$, ½ $Ca^{++}$, ½ $Mg^{++}$, ½ $Zn^{++}$, or 1/3 $Al^{+++}$, preferably $H^+$, $NH_4^+$, $Li^+$, $Na^+$ or $K^+$. |

In at least one embodiment, the optional unit results from the incorporation of a monomer according to formula (A) wherein X is a covalent bond or is $CH_2$. In at least one embodiment, the optional unit results from the incorporation of a monomer according to formula (A) wherein Y is a covalent bond, $CH_2$, C(O)O, or C(O)$NR^3$. In at least one embodiment, the optional unit results from the incorporation of a monomer according to formula (A) wherein M is a covalent bond, -[C(O)O-$CH_2$-$CH_2$]$_n$-, a linear or branched alkylene group with 1 to 6 carbon atoms. In at least one embodiment, the optional unit results from the incorporation of a monomer according to formula (A) wherein $R^1$ is H, methyl or ethyl; X is a covalent bond or is $CH_2$; Y is a covalent bond, $CH_2$, C(O)O, or C(O)$NR^3$; $R^3$ is H, methyl or ethyl; M is a covalent bond, -[C(O)O-$CH_2$-$CH_2$]$_n$-, a linear or branched alkylene group with 1 to 6 carbon atoms; $Z^+$ is $H^+$, $NH_4^+$, $Li^+$, $Na^+$, $K^+$, ½ $Ca^{++}$, ½ $Mg^{++}$, ½ $Zn^{++}$, or 1/3 $Al^{+++}$, or combinations thereof.

In at least one embodiment, the optional unit results from the incorporation of a monomer selected from the group consisting of N-vinylformamide, N-vinylacetamide, N methyl-N-vinylformamide, N-methyl-N-vinylacetamide, N-vinyl-2-pyrrolidone (NVP), N vinylcaprolactam, vinylacetate, methylvinylether, ethylvinylether, methylallylether, ethylmethallylether, styrol, acetoxystyrol, methylmethallylether, ethylallylether, tert-butylacrylamide, N,N-diethylacrylamide, N,N-dimethylacrylamide, N,N-dimethylmethacrylamide, N,N-dipropylacrylamide, N-isopropylacrylamide, N-propylacrylamide, acrylamide, methacrylamide, methylacrylate, methymethylacrylate, tert-butylacrylate, tert-butylmethacrylate, n-butylacrylate, n-butylmethacrylate, laurylacrylate, laurylmethacrylate, behenylacrylate, behenylmethacrylate, cetylacrylate, cetylmethacrylate, stearylacrylate, stearylmethacrylate, tridecylacrylate, tridecylmethacrylate, polyethoxy-(5)-methacrylate, polyethoxy-(5)-acrylate, polyethoxy-(10)-methacrylate, polyethoxy-(10)-acrylate, behenylpolyethoxy-(7)-methacrylate, behenylpolyethoxy-(7)-acrylate, behenylpolyethoxy-(8)-methacrylate, behenylpolyethoxy-(8)-acrylate, behenylpolyethoxy-(12)-methacrylate, behenylpoly-ethoxy-(12)-acrylate, behenylpolyethoxy-(16)-methacrylate, behenylpolyethoxy-(16)-acrylate, behenylpolyethoxy-(25)-methacrylate, behenylpolyethoxy-(25)-acrylate, laurylpoly-ethoxy-(7)-methacrylate, laurylpolyethoxy-(7)-acrylate, laurylpolyethoxy-(8)-methacrylate, laurylpolyethoxy-(8)-acrylate, laurylpolyethoxy-(12)-methacrylate, laurylpolyethoxy-(12)-acrylate, laurylpolyethoxy-(16)-methacrylate, laurylpolyethoxy-(16)-acrylate, laurylpolyethoxy-(22)-methacrylate, laurylpolyethoxy-(22)-acrylate, laurylpolyethoxy-(23)-methacrylate, laurylpolyethoxy-(23)-acrylate, cetylpolyethoxy-(2)-methacrylate, cetylpolyethoxy-(2)-acrylate, cetylpolyethoxy-(7)-methacrylate, cetylpolyethoxy-(7)-acrylate, cetylpolyethoxy-(10)-methacrylate, cetylpolyethoxy-(10)-acrylate, cetylpolyethoxy-(12)-methacrylate, cetylpolyethoxy-(12)-acrylate cetylpoly-ethoxy-(16)-methacrylate, cetylpolyethoxy-(16)-acrylate cetylpolyethoxy-(20)-methacrylate, cetylpolyethoxy-(20)-acrylate, cetylpolyethoxy-(25)-methacrylate, cetylpolyethoxy-(25)-acrylate, cetylpolyethoxy-(25)-methacrylate, cetylpolyethoxy-(25)-acrylate, stearylpolyethoxy-(7)-methacrylate, stearylpolyethoxy-(7)-acrylate, stearylpoly-ethoxy-(8)-methacrylate, stearylpolyethoxy-(8)-acrylate, stearylpolyethoxy-(12)-methacrylate, stearylpolyethoxy-(12)-acrylate, stearylpolyethoxy-(16)-methacrylate, stearylpolyethoxy-(16)-acrylate, stearylpolyethoxy-(22)-methacrylate, stearylpoly-ethoxy-(22)-acrylate, stearylpolyethoxy-(23)-methacrylate, stearylpolyethoxy-(23)-acrylate, stearylpolyethoxy-(25)-methacrylate, stearylpolyethoxy-(25)-acrylate, tridecylpolyethoxy-(7)-methacrylate, tridecylpolyethoxy-(7)-acrylate, tridecylpolythoxy-(10)-methacrylate, tridecylpolyethoxy-(10)-acrylate, tridecylpolyethoxy-(12)-methacrylate, tridecylpolyethoxy-(12)-acrylate, tridecylpolyethoxy-(16)-methacrylate, tridecylpolyethoxy-(16)-acrylate, tridecylpolyethoxy-(22)-methacrylate, tridecylpolyethoxy-(22)-acrylate, tridecylpolyethoxy-(23)-methacrylate, tridecylpolyethoxy-(23)-acrylate, tridecylpolyethoxy-(25)-methacrylate, tridecylpolyethoxy-(25)-acrylate, methoxypolyethoxy-(7)-methacrylate, methoxy-polyethoxy-(7)-acrylate, methoxypoly-ethoxy-(12)-methacrylate, methoxypolyethoxy-(12)-acrylate, methoxypolyethoxy-(16)-methacrylate, methoxypolyethoxy-(16)-acrylate, methoxypolyethoxy-(25)-methacrylate, methoxy-polyethoxy-(25)-acrylate, acrylic acid, ammonium acrylate, sodium acrylate, potassium acrylate, lithium acrylate, zinc acrylate, calcium acrylate, magnesium acrylate, zirconium acrylate, methacrylic acid, ammonium methacrylate, sodium methacrylate, potassium methacrylate, lithium methacrylate, calcium methacrylate, magnesium methacrylate, zirconium methacrylate, zinc methacrylate, 2-carboxyethylacrylate, ammonium 2-carboxyethylacrylate, sodium 2-carboxyethylacrylate, potassium 2-carboxyethylacrylate, lithium 2 carboxyethylacrylate, zinc 2-carboxyethylacrylate, calcium 2-carboxyethylacrylate, magnesium 2-carboxyethylacrylate, zirconium 2-carboxyethylacrylate, 2-carboxyethylacrylate-oligomere, ammonium 2-carboxyethylacrylate-oligomers, sodium 2-carboxyethylacrylate-oligomers, potassium 2-carboxyethylacrylate-oligomers, lithium 2 carboxyethylacrylate-oligomers, zinc 2-carboxyethylacrylate-oligomers, calcium 2-carboxyethylacrylate-oligomers, magnesium 2-carboxyethylacrylate-oligomers, zirconium 2-carboxyethylacrylate-oligomers, itaconic acid , sodium itaconate, potassium itaconate, lithium itaconate, calcium itaconate, magnesium itaconate, zirconium itaconate, zinc itaconate,2-ethylacryl acid, ammonium 2-ethylacrylate, sodium 2-ethylacrylate, potassium 2-ethylacrylate, lithium 2-ethylacrylate, calcium 2-ethylacrylate, magnesium 2-ethylacrylate, zirconium 2-ethylacrylate, zinc 2-ethylacrylate, 2-propylacryl acid, ammonium 2-propylacrylate, sodium 2-propylacrylate, potassium 2-propylacrylate, lithium 2-propylacrylate, calcium 2-propylacrylate, magnesium 2-propylacrylate, magnesium 2-propylacrylate, zirconium 2-propylacrylate, zinc 2-propylacrylate, glycerin propoxylate triacrylate (GPTA), trimethylolpropane triacrylate (TMPTA), pentaerythritoldiacrylate monostearate (PEAS), polyethyleneglycol diacrylate, hexanediol diacrylate (HDDA), hexanediol dimethacrylate (HDDMA), and combinations thereof.

In a preferred embodiment, the optional unit results from the incorporation of a monomer selected from the group consisting of glycerine propoxylate triacrylate (GPTA) and trimethylolpropantriacrylate (TMPTA).

In a preferred embodiment, the optional unit results from the incorporation of a monomer selected from the group consisting of N-vinylformamide, N-vinylacetamide, N-methyl-N-vinylacetamide, N-vinyl-2-pyrrolidone (NVP), N,N-diethylacrylamide, acrylamide, methacrylamide, methylacrylate, methylmethylacrylate, tert-Butylacrylate, acrylic acid, methacrylic acid, 2-carboxyethylacrylate, 2-carboxyethylacrylate oligomers, itaconic acid glycerine propoxylate triacrylate (GPTA), trimethylolpropane triacrylate (TMPTA), pentaerythritol diacrylate monostearate (PEAS) and polyethyleneglycol diacrylate.

In at least one embodiment, the optional unit results from the incorporation of a monomer selected from the group consisting of acrylic acid, methacrylic acid, styrenesulfonic acid, maleic acid, fumaric acid, crotonic acid, itaconic acid, and senecic acid. In at least one embodiment, the optional unit results from monomers selected from the group consisting of open-chain N-vinyl amides, preferably N-vinylformamide (VIFA), N-vinylmethylformamide, N-vinylmethylacetamide (VIMA) and N-vinylacetamides; cyclic N-vinyl amides (N-vinyl lactams) with a ring size of 3 to 9, preferably N-vinylpyrrolidones (NVP) and N-vinylcaprolactam; amides of acrylic and methacrylic acid, preferably acrylamide, methacrylamide, N,N-dimethylacrylamide, N,N-diethylacrylamide, and N,N-diisopropylacrylamide; alkoxylated acrylamides and methacrylamides, preferably hydroxyethyl methacrylate, hydroxymethylmethacrylamide; hydroxyethylmethacryl amide, hydroxypropylmethacrylamide, and mono[2-(methacryloyloxy)ethyl]succinate; N,N-dimethylaminomethacrylate; diethylaminomethylmethacrylate; acrylamideo- and methacrylamideoglycolic acid; 2- and 4-vinylpyridine; vinyl acetate; glycidyl methacrylate; styrene; acrylonitrile; vinyl chloride; stearyl acrylate; lauryl methacrylate; vinylidene chloride; tetrafluoroethylene; and combinations thereof.

Example embodiments of the first aspect

A preferred embodiment of the first aspect relates to a polymer consisting of:

(a) from 9.49 mol-% to 98 mol-%, preferably from 27.5 mol-% to 97.4 mol-% repeating units (a) resulting from the incorporation of acryloyldimethyltaurate wherein at least 10 wt.-%, preferably at least 20 wt.-% of the repeating units resulting from the incorporation of acryloyldimethyltaurate comprise from 28 wt.-% to 100 wt.-% bio-based carbon content, relative to the total mass of carbon in the repeating unit (a) resulting from the incorporation of acryloyldimethyltaurate, measured according to standard ASTM D6866-12, Method B;

wherein the acryloyldimethyltaurate is prepared from acrylonitrile, isobutene and a mixture of sulfuric acid and fuming sulfuric acid comprising sulfur trioxide, wherein at least one of acrylonitrile and isobutene is of bio-based origin, and
wherein the sulfonic acid groups of the repeating units resulting from the incorporation of acryloyldimethyltaurate comprise counterions $Q^+$,
wherein Q+ is H+, NH4+, organic ammonium ions [NHR5R6R7]+ wherein R5, R6, and R7 independently of one another may be hydrogen, a linear or branched alkyl group having 1 to 22 carbon atoms, a linear or branched, singularly or multiply unsaturated alkenyl group having 2 to 22 carbon atoms, a C6-C22 alkylamidopropyl group, a linear mono-hydroxyalkyl group having 2 to carbon atoms or a linear or branched dihydroxyalkyl group having 3 to carbon atoms, and where at least one of the radicals R5, R6, and R7 is not hydrogen, or Q+ is Li+, Na+, K+, ½ Ca++, ½ Mg++, ½ Zn++, 1/3 Al+++, or combinations thereof;

(b) from 0.01 mol-% to 5 mol-%, preferably from 0.01 mol-% to 4 mol-% crosslinking or branching units, wherein the crosslinking or branching units result from the incorporation of a monomer comprising at least two olefinically unsaturated double bonds;

(c) from 0.01 mol-% to 88.52 mol-%, preferably from 0.05 mol-% to 72.4 mol-% of repeating neutral structural units, preferably wherein at least 10 wt.-%, preferably at least 20 wt.-% of the repeating neutral structural units comprise from 0 wt.-% to 100 wt.-% bio-based carbon content, relative to the total mass of carbon in the repeating neutral structural unit, measured according to standard ASTM D6866-12, Method B;

(d) from 1.98 mol-% to 20 mol-%, preferably from 2.5 mol-% to 18 mol-% of repeating anionic structural units, wherein the repeating anionic structural units result from the incorporation of a monomer comprising at least one carboxylate anion, and wherein the repeating anionic structural units are different from units (a), preferably wherein at least 10 wt.-%, preferably at least 20 wt.-% of the repeating anionic structural units comprise from 0 wt.-% to 100 wt.-% bio-based carbon content, relative to the total mass of carbon in the repeating anionic structural unit, measured according to standard ASTM D6866-12, Method B.

A preferred embodiment of the first aspect relates to a polymer comprising: (a) from 9.49 mol-% to 98 mol-%, preferably from 27.5 mol-% to 97.4 mol-% of a repeating structural unit resulting from the incorporation of ACDMT, wherein the ACDMT comprises from 35 wt.-%, preferably from 40 wt.-%, more preferably from 54 wt.-%, even more preferably from 57 wt.-% to 100 wt.-%, most preferably 100 wt.-% bio-based carbon content, relative to the total mass of carbon in ACDMT, measured according to standard ASTM D6866-12, Method B.

SECOND ASPECT

A second aspect relates to a process for obtaining a polymer by polymerization of:

(a) at least one acryloyldimethyltaurate monomer according Formula (10) comprising from 28 wt.-% to 100 wt.-% bio-based carbon content, relative to the total mass of carbon in the acryloyldimethyltaurate monomer according to Formula (10), measured according to standard ASTM D6866-12, Method B; (b) at least one crosslinking or branching monomer; (c) at least one neutral monomer; and (d) at least one anionic monomer;

wherein the crosslinking or branching monomer has at least two olefinically unsaturated double bonds;
and wherein Formula (10) is:

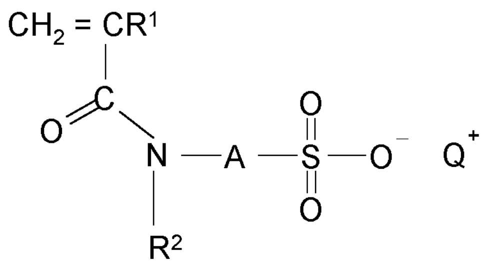

(10)

wherein:

$R^1$ and $R^2$ are H; A is $-C(CH_3)_2-H_2C-$; and

wherein the acryloyldimethyltaurate monomer is prepared from acrylonitrile, isobutene and a mixture of sulfuric acid and fuming sulfuric acid comprising sulfur trioxide, wherein at least one of acrylonitrile and isobutene is of bio-based origin; and

wherein $Q^+$ is $H^+$, $NH_4^+$, organic ammonium ions $[NHR^5R^6R^7]^+$ wherein $R^5$, $R^6$, and $R^7$ independently of one another may be hydrogen, a linear or branched alkyl group having 1 to 22 carbon atoms, a linear or branched, singularly or multiply unsaturated alkenyl group having 2 to 22 carbon atoms, a $C_6$-$C_{22}$ alkylamidopropyl group, a linear mono-hydroxyalkyl group having 2 to carbon atoms or a linear or branched dihydroxyalkyl group having 3 to carbon atoms, and where at least one of the radicals $R^5$, $R^6$, and $R^7$ is not hydrogen, or $Q^+$ is $Li^+$, $Na^+$, $K^+$, ½ $Ca^{++}$, ½ $Mg^{++}$, ½ $Zn^{++}$, 1/3 $Al^{+++}$, or combinations thereof.

In at least one embodiment, the process for obtaining a polymer by polymerization of: (a) at least one acryloyldimethyltaurate monomer according Formula (10) comprising from 28 wt.-% to 100 wt.-% bio-based carbon content, relative to the total mass of carbon in the acryloyldimethyltaurate monomer according to Formula (10), measured according to standard ASTM D6866-12, Method B; (b) at least one crosslinking or branching monomer; (c) at least one neutral monomer; (d) at least one anionic monomer; and (e) at least one optional monomer; wherein the crosslinking or branching monomer has at least two olefinically unsaturated double bonds.

In at least one embodiment, the polymer is according to the first aspect.

Monomer (a)

In at least one embodiment, at least 25 wt.-%, or at least 30 wt.-%, or at least 35 wt.-%, or at least 40 wt.-%, or at least 45 wt.-%, or at least 50 wt.-%, or at least 55 wt.-%, or at least 60 wt.-%, or at least 65 wt.-%, or at least 70 wt.-%, or at least 75 wt.-%, or at least 80 wt.-%, or at least 85 wt.-%, or at least 90 wt.-%, or at least 95 wt.-%, or at least 96 wt.-%, or at least 97 wt.-%, or at least 98 wt.-%, or at least 99 wt.-%, or at least 99.5 wt.-% of the acryloyldimethyltaurate monomer according Formula (10) comprise from 28 wt.-% to 100 wt.-% bio-based carbon content, relative to the total mass of carbon in the repeating unit resulting from the incorporation of acryloyldimethyltaurate, measured according to standard ASTM D6866-12, Method B. In at least one embodiment, the acryloyldimethyltaurate monomers according Formula (10) comprise from 35 wt.-%, preferably from 40 wt.-%, more preferably from 54 wt.-%, even more preferably from 57 wt.-% to 100 wt.-%, most preferably 100 wt.-% bio-based carbon content, relative to the total mass of carbon in the repeating unit (a) resulting from the incorporation of acryloyldimethyltaurate, measured according to standard ASTM D6866-12, Method B.

In at least one embodiment, the monomer according Formula (10) is a compound according to Formula (3);

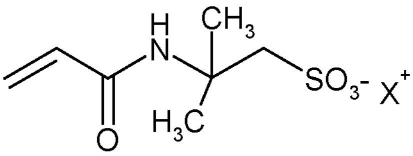

(3)

wherein X is a proton.

In at least one embodiment, the monomer according Formula (10) is ACDMT.

In at least one embodiment, the ACDMT comprises from 35 wt.-%, preferably from 40 wt.-%, more preferably from 54 wt.-%, even more preferably from 57 wt.-% to 100 wt.-%, most preferably 100 wt.-%, bio-based carbon content, relative to the total mass of carbon in ACDMT, measured according to standard ASTM D6866-12, Method B.

Monomer (b)

In at least one embodiment, the polymer comprises from 0.01 mol-% to 10 mol-%, preferably 0.01 mol-% to 5 mol-%, more preferably from 0.01 mol% to 3 mol% of crosslinking or branching units.

In at least one embodiment, the crosslinking or branching units comprise least one oxygen, nitrogen, sulfur or phosphorus atom. In at least one embodiment, the crosslinking or branching units result from monomers having a molecular weight of less than 500 g/mol. In at least one embodiment, the crosslinking or branching units are bifunctional or trifunctional crosslinking agents.

In at least one embodiment, the polymer comprises two or more different crosslinking or branching units.

In at least one embodiment, the crosslinking or branching monomer is according to Formula (2):

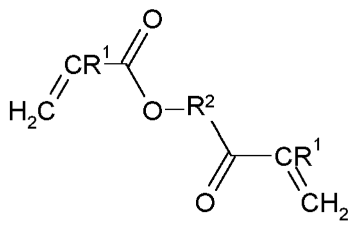

(2)

wherein

$R^1$ is independently selected from H, methyl or ethyl; and
$R^2$ is a linear or branched alkyl group having 1 to 6 carbon atoms, or is a linear or branched, mono- or polyunsaturated alkylene group having 2 to 6 carbon atoms, $-(CH_2-CH_2-O)_n-$
n is a real number between 1 and 100

A monomer according to Formula (2) has the advantage that the polymer can be predicted as a more brush-like polymer. However brush-like polymers show different properties, as linear ones. For example depending on the different comonomer units the solubility could in- or decreased.

In at least one embodiment, the crosslinking or branching monomer is according to Formula (40)

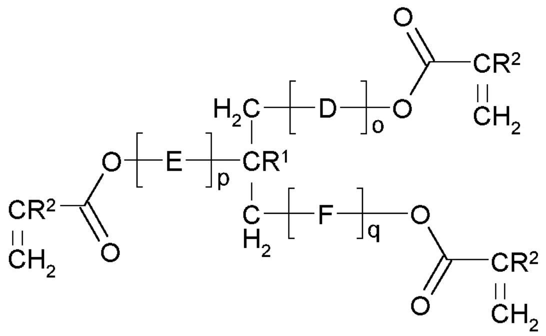

(40)

wherein

$R^1$ is independently selected from H, methyl or ethyl; and
$R^2$ is a linear or branched alkyl group having 1 to 6 carbon atoms, or is a linear or branched, mono- or polyunsaturated alkylene group having 2 to 6 carbon atoms;
D, E, and F are independently methyleneoxy($-CH_2O$), ethyleneoxy($-CH_2-CH_2-O-$), propyleneoxy($-CH(CH_3)-CH_2-O-$), a linear or branched alkylene group having 1 to 6 carbon atoms, a linear or branched, singularly or multiply unsaturated alkenylene group having 2 to 6 carbon atoms, a linear mono-hydroxyalkylene group having 2 to 6 carbon atoms or a linear or branched dihydroxyalkylene group having 3 to 6 carbon atoms; and
o, p, and q each independently are an integer from 1 to 50.

A monomer according to Formula (40) has the advantage that a polymer can be predicted as being highly branched.

In at least one embodiment, the crosslinking or branching monomer is selected from the group consisting of

methylenebisacrylamide; methylenebismethacrylamide; esters of unsaturated monocarboxylic and polycarboxylic acids with polyols, preferably di-acrylates and tri-acrylates and -methacrylates (e.g. glycerol propoxylate triacrylate [GPTA]), more preferably butanediol and ethylene glycol diacrylate and poly ethylene glycol diacrylate and -methacrylate, trimethylolpropane triacrylate (TMPTA) and trimethylolpropane trimethacrylate (TMPTMA); allyl compounds, preferably allyl (meth)acrylate, triallyl cyanurate, diallyl maleate, polyallyl esters, tetraallyloxyethane, triallylamine, tetraallylethylenediamine; allyl esters of phosphoric acid; and/or vinylphosphonic acid derivatives. The choice of crosslinking or branching monomer is important in view of the flexibility of the crosslinks between the main chains of the polymer which affects the final performance of the polymer.

In at least one embodiment, the crosslinking or branching monomer is selected from trimethylolpropane triacrylate (TMPTA) and/or glycerol propoxylate triacrylate (GPTA).

Particularly preferred as crosslinking or branching monomer for the polymers of the invention are glycerol propoxylate triacrylate (GPTA), trimethylolpropane triacrylate (TMPTA), pentaerythritol diacrylate mono stearate (PEAS), hexanediol diacrylate (HDDA), poly ethylene glycol diacrylate (PEG-DA) and hexanediol dimethacrylate (HDDMA). Especially preferred is glycerol propoxylate triacrylate (GPTA) and trimethylolpropane triacrylate (TMPTA).

Monomer (c)

In at least one embodiment, the polymer comprises at least one repeating neutral structural units.

In at least one embodiment, the polymer comprises (c) from 0.99 mol-% to 59.99 mol-%, preferably from 1.99 mol-% to 44.99 mol-% of repeating neutral structural units; wherein the repeating neutral units comprises up to 100 wt.-% bio-based carbon content, relative to the total mass of carbon in the repeating unit, measured according to standard ASTM D6866-12, Method B. In at least one embodiment, at least 25 wt.-%, or at least 30 wt.-%, or at least 35 wt.-%, or at least 40 wt.-%, or at least 45 wt.-%, or at least 50 wt.-%, or at least 55 wt.-%, or at least 60 wt.-%, or at least 65 wt.-%, or at least 70 wt.-%, or at least 75 wt.-%, or at least 80 wt.-%, or at least 85 wt.-%, or at least 90 wt.-%, or at least 95 wt.-%, or at least 96 wt.-%, or at least 97 wt.-%, or at least 98 wt.-%, or at least 99 wt.-%, or at least 99.5 wt.-% of the monomers (c) comprise from 28 wt.-% to 100 wt.-% bio-based carbon content, relative to the total mass of carbon in the repeating unit resulting from the incorporation of acryloyldimethyltaurate, measured according to standard ASTM D6866-12, Method B.

In at least one embodiment, the neutral monomer is selected from the group consisting of N-vinylformamide, N-vinylacetamide, N-methyl-N-vinylformamide, N-methyl-N-vinylacetamide, N-vinyl-2-pyrrolidone, N-vinylcaprolactam, vinylacetate, N,N-dimethylacrylamide, N-isopropylacrylamide, acrylamide, methylacrylate, behenylpoly-ethoxy-(25)-methacrylate, laurylpoly-ethoxy-(7)-methacrylate, cetylpolyethoxy-(10)-methacrylate, stearylpoly-ethoxy-(8)-methacrylate, methoxypoly-ethoxy-(12)-methacrylate, and combinations thereof.

Monomer (d)

In at least one embodiment, the polymer comprises at least one repeating anionic structural unit.

In at least one embodiment, the polymer comprises from 1.98 mol-% to 20 mol-%, preferably from 2.5 mol-% to 18 mol-% of repeating anionic structural units, wherein the repeating anionic structural units result from the incorporation of a monomer comprising at least one carboxylate anion, and wherein the repeating anionic structural units are different from units (a) and wherein the repeating anionic structural units comprises up to 100 wt.-% bio-based carbon content, relative to the total mass of carbon in the repeating unit, measured according to standard ASTM D6866-12, Method B. In at least one embodiment, at least 25 wt.-%, or at least 30 wt.-%, or at least 35 wt.-%, or at least 40 wt.-%, or at least 45 wt.-%, or at least 50 wt.-%, or at least 55 wt.-%, or at least 60 wt.-%, or at least 65 wt.-%, or at least 70 wt.-%, or at least 75 wt.-%, or at least 80 wt.-%, or at least 85 wt.-%, or at least 90 wt.-%, or at least 95 wt.-%, or at least 96 wt.-%, or at least 97 wt.-%, or at least 98 wt.-%, or at least 99 wt.-%, or at least 99.5 wt.-% of the monomers (d) comprise from 28 wt.-% to 100 wt.-% bio-based carbon content, relative to the total mass of carbon in the repeating unit resulting from the incorporation of acryloyldimethyltaurate, measured according to standard ASTM D6866-12, Method B.

In at least one embodiment, the anionic monomer (d) is according to formula (A):

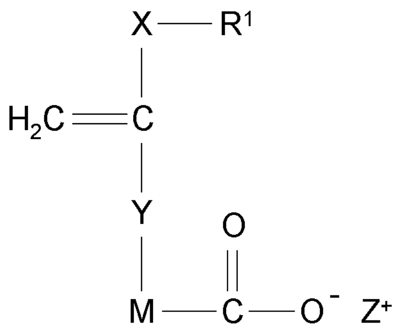

(A)

wherein

| | |
|---|---|
| $R^1$ and $R^3$ | are H, methyl or ethyl, or $C(O)O^-Z^+$; |
| X, Y | are selected from a covalent bond, O, $CH_2$, C(O)O, OC(O), $C(O)NR^3$ or $NR^3C(O)$; |
| M | are selected from a covalent bond, $-[C(O)O-CH_2-CH_2]_n-$, a linear or branched alkylene group with 1 to 6 carbon atoms, a linear or branched, mono- or polyunsaturated alkenylene group with 2 to 6 carbon atoms, a linear mono-hydroxyalkylene group with 2 to 6 carbon atoms or a linear or branched di-hydroxyalkylene group with 3 to 6 carbon atoms; |
| n | is an integer from 1 - 5 and |
| $Z^+$ | is $H^+$, $NH_4^+$, an organic ammonium ion $[HNR^5R^6R^7]^+$ wherein $R^5$, $R^6$ and $R^7$ are independently hydrogen, a linear or branched alkyl group with 1 to 22 carbon atoms, a linear or branched, mono- or polyunsaturated alkenyl group with 2 to 22 carbon atoms, a $C_6$ to $C_{22}$ alkylamidopropyl group, a linear mono-hydroxyalkyl group with 2 to 10 carbon atoms or a linear or branched di-hydroxyalkyl group with 3 to 10 carbon atoms, and wherein at least one of $R^5$, $R^6$ and $R^7$ is not hydrogen, or $Z^+$ is $Li^+$, $Na^+$, $K^+$, ½ $Ca^{++}$, ½ $Mg^{++}$, ½ $Zn^{++}$, 1/3 $Al^{+++}$, or combinations thereof. In at least one embodiment, the $Z^+$ is $H^+$, $NH_4^+$, $Li^+$, $Na^+$, $K^+$, ½ $Ca^{++}$, ½ $Mg^{++}$, ½ $Zn^{++}$, or 1/3 $Al^{+++}$, preferably $H^+$, $NH_4^+$, $Li^+$, $Na^+$ or $K^+$. |

In at least one embodiment, the anionic monomer is selected from the group consisting of acrylic acid or acrylate methacrylic acid or methacrylate, itaconic acid or itaconate, carboxyethylacrylic acid or carboxyethylacrylate, carboxyethylacrylic acid oligomers or carboxyethylacrylate oligomers, 2-propylacrylic acid or 2-propylacrylate, 2-ethylacrylic acid or 2-ethylacrylate, and their respective alkali or alkaline earth metal salts.

In at least one embodiment, the anionic monomer is selected from the group consisting of acrylic acid or acrylate methacrylic acid or methacrylate, itaconic acid or itaconate, carboxyethylacrylic acid or carboxyethylacrylate, carboxyethylacrylic acid oligomers or carboxyethylacrylate oligomers, and their respective alkali or alkaline earth metal salts. These anionic monomers are preferred because they can easily synthesised from bio-based sources.

Optional monomer (e)

In at least one embodiment, the polymer comprises at least one optional unit. In at least one embodiment, the optional monomer is selected from the group consisting of unsaturated carboxylic acids and their anhydrides and salts, and also their esters with aliphatic, olefinic, cycloaliphatic, arylaliphatic or aromatic alcohols having a carbon number of from 1 to 22. In at least one embodiment, the optional monomer is selected from the group consisting of functionalised (meth)acrylic acid esters, acrylic or methacrylic acid amides, polyglycol acrylic or methacrylic acid esters, polyglycol acrylic or methacrylic acid amides, dipropyleneglycolacrylic or methacrylic acid esters, dipropylenglycolacrylic or methacrylic acid amides, ethoxylated fatty alcohol acrylates or -methacrylates, propoxylated fatty alcohol acrylates or linear or cyclic N-vinylamides or N-methylvinyl amides.

In at least one embodiment, the optional monomer is according to formula (A):

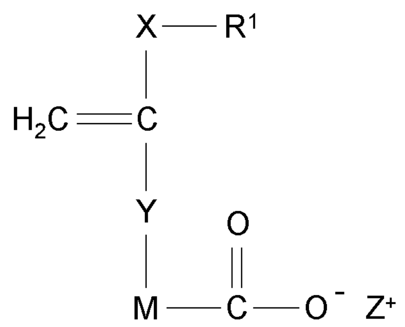

(A)

wherein:

| | |
|---|---|
| X, Y | are selected from a covalent bond, O, $CH_2$, C(O)O, OC(O), C(O)$NR^3$ or $NR^3$C(O); |
| $R^1$ and $R^3$ | are H, methyl or ethyl, or C(O)$O^-Z^+$; |
| M | is selected from a covalent bond, -[C(O)O-$CH_2$-$CH_2$]$_n$-, a linear or branched alkylene group with 1 to 6 carbon atoms, a linear or branched, mono- or polyunsaturated alkenylene group with 2 to 6 carbon atoms, a linear mono-hydroxyalkylene group with 2 to 6 carbon atoms or a linear or branched di-hydroxyalkylene group with 3 to 6 carbon atoms; |
| n | is an integer from 1 - 5, and |
| $Z^+$ | is $H^+$, $NH_4^+$, an organic ammonium ion $[HNR^5R^6R^7]^+$ wherein $R^5$, $R^6$ and $R^7$ are independently hydrogen, a linear or branched alkyl group with 1 to 22 carbon atoms, a linear or branched, mono- or polyunsaturated alkenyl group with 2 to 22 carbon atoms, a $C_6$ to $C_{22}$ alkylamidopropyl group, a linear mono-hydroxyalkyl group with 2 to 10 carbon atoms or a linear or branched di-hydroxyalkyl group with 3 to 10 carbon atoms, and wherein at least one of $R^5$, $R^6$ and $R^7$ is not hydrogen, or $Z^+$ is $Li^+$, $Na^+$, $K^+$, ½ $Ca^{++}$, ½ $Mg^{++}$, ½ $Zn^{++}$, 1/3 $Al^{+++}$, or combinations thereof. In at least one embodiment, the $Z^+$ is $H^+$, $NH_4^+$, $Li^+$, $Na^+$, $K^+$, ½ $Ca^{++}$, ½ $Mg^{++}$, ½ $Zn^{++}$, or 1/3 $Al^{+++}$, preferably $H^+$, $NH_4^+$, $Li^+$, $Na^+$ or $K^+$. |

In at least one embodiment, the optional monomer is according to formula (A) wherein X is a covalent bond or is $CH_2$. In at least one embodiment, the optional monomer is according to formula (A) wherein Y is a covalent bond, $CH_2$, C(O)O, or C(O)$NR^3$. In at least one embodiment, the optional monomer is according to formula (A) wherein M is a covalent bond, -[C(O)O-$CH_2$-$CH_2$]$_n$-, a linear or branched alkylene group with 1 to 6 carbon atoms. In at least one embodiment, the optional monomer is according to formula (A) wherein $R^1$ is H, methyl or ethyl; X is a covalent bond or is $CH_2$; Y is a covalent bond, $CH_2$, C(O)O, or C(O)$NR^3$; $R^3$ is H, methyl or ethyl; M is a covalent bond, -[C(O)O-$CH_2$-$CH_2$]$_n$-, a linear or branched alkylene group with 1 to 6 carbon atoms; $Z^+$ is $H^+$, $NH_4^+$, $Li^+$, $Na^+$, $K^+$, ½ $Ca^{++}$, ½ $Mg^{++}$, ½ $Zn^{++}$, or 1/3 $Al^{+++}$, or combinations thereof.

In at least one embodiment, the optional monomer (e) is selected from the group consisting of N-vinylformamide, N-vinylacetamide, N methyl-N-vinylformamide, N-methyl-N-vinylacetamide, N-vinyl-2-pyrrolidone (NVP), N vinylcaprolactam, vinylacetate, methylvinylether, ethylvinylether, methylallylether, ethylmethallylether, styrol, acetoxystyrol, methylmethallylether, ethylallylether, tert-butylacrylamide, N,N-diethylacrylamide, N,N-dimethylacrylamide, N,N-dimethylmethacrylamide, N,N-dipropylacrylamide, N-isopropylacrylamide, N-propylacrylamide, acrylamide, methacrylamide, methylacrylate, methymethylacrylate, tert-butylacrylate, tert.-butylmethacrylate, n-butylacrylate, n-butylmethacrylate, laurylacrylate, laurylmethacrylate, behenylacrylate, behenylmethacrylate, cetylacrylate, cetylmethacrylate, stearylacrylate, stearylmethacrylate, tridecylacrylate, tridecylmethacrylate, polyethoxy-(5)-methacrylate, polyethoxy-(5)-acrylate, polyethoxy-(10)-methacrylate, polyethoxy-(10)-acrylate, behenylpolyethoxy-(7)-methacrylate, behenylpolyethoxy-(7)-acrylate, behenylpolyethoxy-(8)-methacrylate, behenylpoly-ethoxy-(8)-acrylate, behenylpolyethoxy-(12)-methacrylate, behenylpoly-ethoxy-(12)-acrylate, behenylpolyethoxy-(16)-methacrylate, behenylpolyethoxy-(16)-acrylate, behenylpolyethoxy-(25)-methacrylate, behenylpolyethoxy-(25)-acrylate, laurylpolyethoxy-(7)-methacrylate, laurylpolyethoxy-(7)-acrylate, laurylpolyethoxy-(8)-methacrylate, laurylpolyethoxy-(8)-acrylate, laurylpolyethoxy-(12)-methacrylate, laurylpolyethoxy-(12)-acrylate, laurylpolyethoxy-(16)-methacrylate, laurylpolyethoxy-(16)-acrylate, laurylpolyethoxy-(22)-methacrylate, laurylpolyethoxy-(22)-acrylate, laurylpolyethoxy-(23)-methacrylate, laurylpolyethoxy-(23)-acrylate, cetylpolyethoxy-(2)-methacrylate, cetylpolyethoxy-(2)-acrylate, cetylpolyethoxy-(7)-methacrylate, cetylpolyethoxy-(7)-acrylate, cetylpolyethoxy-(10)-methacrylate, cetylpolyethoxy-(10)-acrylate, cetylpolyethoxy-(12)-methacrylate, cetylpolyethoxy-(12)-acrylate cetylpoly-ethoxy-(16)-methacrylate, cetylpolyethoxy-(16)-acrylate cetylpolyethoxy-(20)-methacrylate, cetylpolyethoxy-(20)-acrylate, cetylpolyethoxy-(25)-methacrylate, cetylpolyethoxy-(25)-acrylate, cetylpolyethoxy-(25)-methacrylate, cetylpolyethoxy-(25)-acrylate, stearylpolyethoxy-(7)-methacrylate, stearylpolyethoxy-(7)-acrylate, stearylpoly-ethoxy-(8)-methacrylate, stearylpolyethoxy-(8)-acrylate, stearylpolyethoxy-(12)-methacrylate, stearylpolyethoxy-(12)-acrylate, stearylpolyethoxy-(16)-methacrylate, stearylpolyethoxy-(16)-acrylate, stearylpolyethoxy-(22)-methacrylate, stearylpoly-ethoxy-(22)-acrylate, stearylpoly-ethoxy-(23)-methacrylate, stearylpolyethoxy-(23)-acrylate, stearylpolyethoxy-(25)-methacrylate, stearylpoly-ethoxy-(25)-acrylate, tridecylpolyethoxy-(7)-methacrylate, tridecylpolyethoxy-(7)-acrylate, tridecylpolythoxy-(10)-methacrylate, tridecylpolyethoxy-(10)-acrylate, tridecylpolyethoxy-(12)-methacrylate, tridecylpolyethoxy-(12)-acrylate, tridecylpolyethoxy-(16)-methacrylate, tridecylpolyethoxy-(16)-acrylate, tridecylpolyethoxy-(22)-methacrylate, tridecylpoly-ethoxy-(22)-acrylate, tridecylpolyethoxy-(23)-methacrylate, tridecylpolyethoxy-(23)-acrylate, tridecylpoly-ethoxy-(25)-methacrylate, tridecylpolyethoxy-(25)-acrylate, methoxypolyethoxy-(7)-methacrylate, methoxy-polyethoxy-(7)-acrylate, methoxypoly-ethoxy-(12)-methacrylate, methoxypolyethoxy-(12)-acrylate, methoxypoly-ethoxy-(16)-methacrylate, methoxypolyethoxy-(16)-acrylate, methoxypolyethoxy-(25)-methacrylate, methoxy-polyethoxy-(25)-acrylate, acrylic acid, ammonium acrylate, sodium acrylate, potassium acrylate, lithium acrylate, zinc acrylate, calcium acrylate, magnesium acrylate, zirconium acrylate, methacrylic acid, ammonium methacrylate, sodium methacrylate, potassium methacrylate, lithium methacrylate, calcium methacrylate, magnesium methacrylate, zirconium methacrylate, zinc methacrylate, 2-carboxyethylacrylate, ammonium 2-carboxyethylacrylate, sodium 2-carboxyethylacrylate, potassium 2-carboxyethylacrylate, lithium 2 carboxyethylacrylate, zinc 2-carboxyethylacrylate, calcium 2-carboxyethylacrylate, magnesium 2-carboxyethylacrylate, zirconium 2-carboxyethylacrylate, 2-carboxyethylacrylate-oligomere, ammonium 2-carboxyethylacrylate-oligomers, sodium 2-carboxyethylacrylate-oligomers, potassium 2-carboxyethylacrylate-oligomers, lithium 2 carboxyethylacrylate-oligomers, zinc 2-carboxyethylacrylate-oligomers, calcium 2-carboxyethylacrylate-oligomers, magnesium 2-carboxyethylacrylate-oligomers, zirconium 2-carboxyethylacrylate-oligomers, itaconic acid , sodium itaconate, potassium itaconate, lithium itaconate, calcium itaconate, magnesium itaconate, zirconium itaconate, zinc itaconate,2-ethylacryl acid, ammonium 2-ethylacrylate, sodium 2-ethylacrylate, potassium 2-ethylacrylate, lithium 2-ethylacrylate, calcium 2-ethylacrylate, magnesium 2-ethylacrylate, zirconium 2-ethylacrylate, zinc 2-ethylacrylate, 2-propylacryl acid, ammonium 2-propylacrylate, sodium 2-propylacrylate, potassium 2-propylacrylate, lithium 2-propylacrylate, calcium 2-propylacrylate, magnesium 2-propylacrylate, magnesium 2-propylacrylate, zirconium 2-propylacrylate, zinc 2-propylacrylate, glycerin propoxylate triacrylate (GPTA), trimethylolpropane triacrylate (TMPTA), pentaerythritoldiacrylate monostearate (PEAS), polyethyleneglycol diacrylate, hexanediol diacrylate (HDDA), hexanediol dimethacrylate (HDDMA), and combinations thereof.

In a preferred embodiment, the optional monomer (e) is selected from the group consisting of glycerine propoxylate triacrylate (GPTA) and trimethylolpropantriacrylate (TMPTA).

In a preferred embodiment, the optional monomer (e) is selected from the group consisting of N-vinylformamide, N-vinylacetamide, N-methyl-N-vinylacetamide, N-vinyl-2-pyrrolidone (NVP), N,N-diethylacrylamide, acrylamide, methacrylamide, methylacrylate, methylmethylacrylate, tert-Butylacrylate, acrylic acid, methacrylic acid, 2-carboxyethylacrylate, 2-carboxyethylacrylate oligomers, itaconic acid glycerine propoxylate triacrylate (GPTA), trimethylolpropane triacrylate (TMPTA), pentaerythritol diacrylate monostearate (PEAS) and polyethyleneglycol diacrylate.

In at least one embodiment, the optional monomer (e) is selected from the group consisting of acrylic acid, methacrylic acid, styrenesulfonic acid, maleic acid, fumaric acid, crotonic acid, itaconic acid, and senecic acid. In at least one embodiment, the optional monomer is selected from the group consisting of open-chain N-vinyl amides, preferably N-vinylformamide (VIFA), N-vinylmethylformamide, N-vinylmethylacetamide (VIMA) and N-vinylacetamides; cyclic N-vinyl amides (N-vinyl lactams) with a ring size of 3 to 9, preferably N-vinylpyrrolidones (NVP) and N-vinylcaprolactam; amides of acrylic and methacrylic acid, preferably acrylamide, methacrylamide, N,N-dimethylacrylamide, N,N-diethylacrylamide, and N,N-diisopropylacrylamide; alkoxylated acrylamides and methacrylamides, preferably hydroxyethyl methacrylate, hydroxymethylmethacrylamide; hydroxyethylmethacryl amide, hydroxypropylmethacrylamide, and mono[2-(methacryloyloxy)ethyl]succinate; N,N-dimethylaminomethacrylate; diethylaminomethylmethacrylate; acrylamideo- and methacrylamideoglycolic acid; 2- and 4-vinylpyridine; vinyl acetate; glycidyl methacrylate; styrene; acrylonitrile; vinyl chloride; stearyl acrylate; lauryl methacrylate; vinylidene chloride; tetrafluoroethylene; and combinations thereof.

Polymerisation

In at least one embodiment, the above monomers are dissolved or dispersed in a polar solvent. The polymerisation is preferably initiated by the addition of a radical building compound.

In at least one embodiment, the acryloyldimethyltaurate monomer according Formula (10) is neutralised with a base prior to polymerisation.

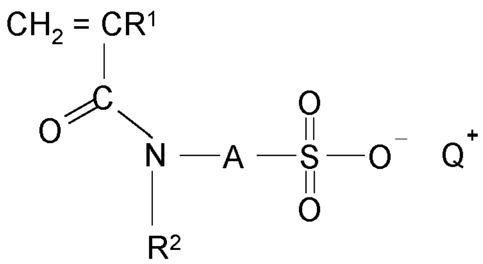

(10)

In at least one embodiment, the acryloyldimethyltaurate monomer according formula (10) is neutralized following polymerization using a base. In the acryloyldimethyltaurate monomer according to Formula (10), $R^1$ and $R^2$ are H; A is $-C(CH_3)_2-H_2C-$; and $Q^+$ is a cation.

In at least one embodiment, in Formula (10) $Q^+$ is $H^+$, $NH_4^+$, morpholine, an organic ammonium ion $[NHR^5R^6R^7]^+$ wherein $R^5$, $R^6$, and $R^7$ independently of one another is hydrogen, a linear or branched alkyl group having 1 to 22 carbon atoms, a linear or branched, mono- or poly-unsaturated alkenyl group having 2 to 22 carbon atoms, a $C_6$-$C_{22}$ alkylamidopropyl group, a linear mono-hydroxyalkyl group having 2 to 10 carbon atoms or a linear or branched dihydroxyalkyl group having 3 to 15 carbon atoms, and wherein at least one of the radicals $R^5$, $R^6$, and $R^7$ is not hydrogen, or $X^+$ is $Li^+$, $Na^+$, $K^+$, ½ $Ca^{++}$, ½ $Mg^{++}$, ½ $Zn^{++}$, 1/3 $Al^{+++}$, or combinations thereof. Preferably $Q^+$ is $H^+$, $NH_4^+$ or $Na^+$. Most preferably, $Q^+$ is $Na^+$ and $NH_4^+$. In at least one embodiment, $Q^+$ is $NH_4^+$. In at least one embodiment, $Q^+$ is selected from the group monoalkylammonium, dialkylammonium, trialkylammonium and/or tetraalkylammonium salts, in which the alkyl substituents of the amines may independently of one another be ($C_1$ to $C_{22}$)-alkyl radicals or ($C_2$ to $C_{10}$)-hydroxyalkyl radicals.

The monomer according to Formula (10) is acryloyldimethyltaurate.

In at least one embodiment, the acryloyldimethyltaurate monomer according to Formula (10) has a degree of neutralisation of between 0 mol-% and 100 mol-%. In at least one embodiment, the acryloyldimethyltaurate monomer according to Formula (10) has a degree of neutralisation of from 50.0 to 100 mol-%, preferably from 80 mol-% to 100 mol-%, more preferably from 90.0 to 100 mol-%, even more preferably from 95.0 to 100 mol-%. Particular preference being given to a degree of neutralisation of more than 80 mol-%, more preferably more than 90 mol-%, even more preferably more than 95 mol-%.

In at least one embodiment, the acryloyldimethyltaurate monomer according to Formula (10) can be neutralized by using gaseous ammonia, ammonia hydroxide solution, morpholine, monoalkylammine, dialkylammine, trialkylammine, tetraalkylammonium salts, sodium hydrogen carbonate, sodium carbonate, sodium hydroxide, potassium hydrogen carbonate, potassium carbonate, potassium hydroxide, lithium hydrogen carbonate, lithium carbonate, lithium hydroxide, preferably gaseous ammonia, morpholine, and sodium hydrogen carbonate.

In at least one embodiment, the synthesis of the polymer is carried out by radical precipitation polymerization in a polar solvent or a polar solvent mixture. Preferably the polar solvent or a polar solvent mixture has a boiling point between 60 °C and 110 °C, preferably between 60 °C and 95 °C and more preferably between 60 °C and 95 °C.

In at least one embodiment, the radical precipitation polymerization is carried out in a polar solvent mixture comprising:

I) water and
II) a further compound.

In at least one embodiment the compound II) is polar and organic.

In at least one embodiment the compound II) is one or more polar alcohols and one or more ketones.

In a preferred embodiment, the compound II) is selected from the group consisting methanol, ethanol, 1-propanol, 2-propanol, 2-methyl-2-propanol, 1-butanol, 2-butanol, dimethyl ketone, diethyl ketone, pentan-2-one, butanone, tetrahydro pyrane, tetrahydro furane, 2-methyl-tetrahydro furane, 1,3-dioxane, 1,4-dioxane, preferably 2-propanol, 2-methyl-2-propanol, dimethyl ketone, tetrahydro furane, 2-methyl-tetrahydro furane, more preferably 2-methyl-2-propanol and dimethyl ketone.

In a preferred embodiment, the solvent mixture contains from 0.5 up to 10 wt.-%, preferably from 1 up to 8 wt.-% and more preferably from 2 up to 5 wt.-% water.

In a preferred embodiment, the solvent mixture contains from 1 up to 99.5 wt.-%, preferably from 5 up to 95 wt.-% and more preferably from 10 up to 90 wt.-% 2-methyl-2-propanol.

In a preferred embodiment, the polar solvent mixture comprises from 0.5 up to 10 wt.-% water, from 1 up to 98.5 wt.-% 2-methyl-2-propanol and from 1 up to 98.5 wt.-% dimethyl ketone, preferably from 0.5 up to 7.5 wt.-% water, from 5 up to 94.5 wt.-% 2-methyl-2-propanol and from 5 up to 94.5 wt.-% dimethyl ketone.

In a preferred embodiment, the polymerization of the monomers (a) to (b), optionally (a) to (c), optionally (a) to (d), optionally (a) to (e), is carried out in a solvent mixture comprising water, 2-methyl-2-propanol and dimethyl ketone. Preferably the water content of the solvent mixture should not be higher as 10 wt.-%, otherwise the synthesized polymer build lumps during the polymerization.

In a preferred embodiment, the polymerization reaction is initiated by a radical building compound. In at least one embodiment, the radical building compound is selected from the group of azo-initiators (e.g. azo-bis-iso butyronitrile, 2,2'-azobis(4-methoxy-2.4-dimethyl valeronitrile), 2,2'-azobis(2.4-dimethyl valeronitrile), dimethyl 2,2'-azobis(2-methylpropionate), 2,2'-azobis(2-methylbutyronitrile), 1,1'-azobis(cyclohexane-1-carbonitrile) or 2,2'-azobis[N-(2-propenyl)-2-methyl-propionamide]), peroxides (e.g. dilauryl peroxide, tert.-butylhydro peroxide, di-tert.-butyl peroxide, tri phenyl methyl hydroperoxid, benzoylperoxid) or persulfates. In at least one embodiment, the initiation of the polymerization starts in a temperature interval between 30 °C up to 80 °C, preferably between 40 °C up to 70 °C during 30 minutes to serial hours.

In at least one embodiment, the polymer has a weight average molecular weight of at least 700 g/mol, preferably from 700 g/mol to 10 million g/mol.

THIRD ASPECT

A third aspect relates to the use of the polymer according to the first aspect as a thickening agent and/or rheology modifier and/or a viscosity modifiers. For example, the thickening agent and/or rheology modifier can be used as an additive in the oil and mining industry e.g. to increase the efficiency of processes for isolating crude oil.

A thickening agent or thickener is a substance which can increase the viscosity of a liquid without substantially changing its other properties. Edible thickeners are commonly used to thicken sauces, soups, and puddings without altering their taste; thickeners are also used in paints, inks, explosives, and cosmetics.

Thickeners may also improve the suspension of other ingredients or emulsions which increases the stability of the product. Thickening agents are often regulated as food additives and as cosmetics and personal hygiene product ingredients. Some thickening agents are gelling agents (gellants), forming a gel, dissolving in the liquid phase as a colloid mixture that forms a weakly cohesive internal structure. Others act as mechanical thixotropic additives with discrete particles adhering or interlocking to resist strain.

Thickening agents can also be used when medical condition such as dysphagia cause individuals difficulty when swallowing. Thickened liquids play a vital role in reducing risk of aspiration for dysphagia patients.

FOURTH ASPECT

A fourth aspect relates to a composition comprising the polymer of the first aspect. In at least one embodiment, the composition comprises at least 0.5 wt.-% of said polymer. In an alternative composition, the composition comprises at least 0.01 wt.-%, or at least 0.05 wt.-%, or at least 0.1 wt.-%, or at least 0.5 wt.-%, or at least 1 wt.-%, or at least 1.5 wt.-%, or at least 2 wt.-%, or at least 2.5 wt.-%, or at least 3 wt.-%, or at least 4 wt.-%, or at least 5 wt.-%, or at least 7.5 wt.-%, or at least 10 wt.-%, or at least 12 wt.-%, or at least 15 wt.-%, or at least 20 wt.-%, or at least 25 wt.-%, or at least 30 wt.-%, or at least 35 wt.-%, or at least 40 wt.-%, or at least 45 wt.-%, or up to 50 wt.-% polymer of the first aspect.

In at least one embodiment, the composition comprises: (I) the polymer according to the first aspect; and (II) a further component.

In at least one embodiment, the further component is a carrier, solvent or diluent. In at least one embodiment, the composition comprises a solvent, wherein the solvent comprises water and/or alcohol. Solvent is useful for providing the compounds used in present invention in liquid form. In at least one embodiment, the solvent is cosmetically acceptable. In at least one embodiment, the composition comprises at least 10 wt.-% water. Water is useful for economic reasons but also because it is cosmetically acceptable. Optionally the composition comprises water-miscible or water-soluble solvents such as lower alkyl alcohols. In at least one embodiment, the composition comprises $C_1$-$C_5$ alkyl monohydric alcohols, preferably $C_2$-$C_3$ alkyl alcohols. The alcohols which may be present are in particular lower monohydric or polyhydric alcohols having 1 to 4 carbon atoms customarily used for cosmetic purposes, such as preferably ethanol and isopropanol. Optionally, the composition comprises a water-soluble polyhydric alcohol. In at least one embodiment, the water-soluble polyhydric alcohols are polyhydric alcohols having two or more hydroxyl groups in the molecule. In at least one embodiment, the water-soluble polyhydric alcohol is selected from the group consisting of: dihydric alcohols such as ethylene glycol, propylene glycol, trimethylene glycol, 1,2-butylene glycol, 1,3-butylene glycol, 1,4-butylene glycol, tetramethylene glycol, 2,3-butylene glycol, pentamethylene glycol, 2-butene-1,4-diol, hexylene glycol, octylene glycol; trihydric alcohols such as glycerine, trimethylol propane, 1,2,6-hexanetriol; tetrahydric alcohols such as penthaerythritol; pentahydric alcohols such as xylytol; hexahydric alcohols such as sorbitol, mannitol; polyhydric alcohol polymers such as diethylene glycol, dipropylene glycol, polyethylene glycol, polypropylene glycol, tetraethylene glycol, diglycerine, polyethylene glycol, triglycerine, tetraglycerine, polyglycerine; dihydric alcohol alkyl ethers such as ethylene glycol monomethyl ether, ethylene glycol monoethyl ether, ethylene glycol monobutyl ether, ethylene glycol monophenyl ether,

ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate; glycerine monoalkyl ethers such as xyl alcohol, selachyl alcohol, batyl alcohol; sugar alcohols such as sorbitol, maltitol, maltotriose, mannitol, sucrose, erythritol, glucose, fructose, starch sugar, maltose, xylytose, starch sugar reduced alcohol, glysolid, tetrahydrofurfuryl alcohol, POE tetrahydrofurfuryl alcohol, POP butyl ether, POP POE butyl ether, tripolyoxypropylene glycerine ether, POP glycerine ether, POP glycerine ether phosphoric acid, POP POE pentanerythritol ether, and mixtures thereof. In a preferred embodiment, the composition comprises a solvent selected from the group consisting of water, glycols, ethanol, and combinations thereof. In a preferred embodiment, the composition comprises an aqueous, alcoholic or aqueous-alcoholic solvent, and wherein the aqueous, alcoholic or aqueous-alcoholic solvent comprises water, ethanol, propanol, isopropanol, 1,2-propylene glycol, 1,3-propylene glycol, isobutanol, butanol, butyl glycol, butyl diglycol, glycerol, or a mixture thereof; preferably wherein the aqueous, alcoholic or aqueous-alcoholic solvent comprises water, ethanol, propanol, isopropanol, 1,2-propylene glycol, 1,3-propylene glycol, glycerol, or mixtures thereof; more preferably wherein the aqueous, alcoholic or aqueous-alcoholic solvent comprises water, isopropanol, 1,2-propylene glycol, 1,3-propylene glycol, or mixtures thereof; even more preferably wherein the aqueous, alcoholic or aqueous-alcoholic solvent consists of water or consists of a mixture of water and an alcohol wherein the alcohol is selected from the group consisting of isopropanol, 1,2-propylene glycol and 1 ,3-propylene glycol. Natural solvents can also be used. In at least one embodiment, the composition comprises a solvent selected from the group consisting of plant oil, honey, plant-derived sugar compositions, and mixtures thereof. In at least one embodiment, the composition comprises from 0.5 wt.-% to 90 wt.-%, preferably from 1.0 wt.-% to 80 wt.-%, even more preferably from 5.0 wt.-% to 70 wt.-% of at least one carrier, solvent and/ or diluent.

In at least one embodiment, the composition comprising a polymer according to the first aspect relates to a coating and/or adhesive composition. The sulfonic acid group of the polymers according to the first aspect gives an ionic character over a wide range of pH. Anionic charges from these polymers fixed on polymer particles enhance the chemical and shear stabilities of polymer emulsion and also reduce the amount of surfactants leaching out of paint film. It improves the thermal and mechanical properties of adhesives, and increases the adhesive strength of pressure-sensitive adhesive formulations.

In at least one embodiment, the composition comprising a polymer according to the first aspect relates to a detergent composition. The polymers according to the first aspect enhances the washing performance of surfactants by binding multivalent cations and reducing dirt attachment.

In at least one embodiment, the composition comprising a polymer according to the first aspect relates to personal care composition. The strong polar and hydrophilic properties of the polymers according to the first aspect are exploited as a very efficient lubricant in skin care compositions. The polymers according to the first aspect can be used in bath & shower composition and in hair care composition.

In at least one embodiment, the composition comprising a polymer according to the first aspect relates to home care composition. The polymers according to the first aspect can show good performance in dish washing compositions, in fabric care and can be used in surface cleaning compositions.

In at least one embodiment, the composition comprising a polymer according to the first aspect relates to a medical hydrogel. The polymers according to the first aspect demonstrate a high water-absorbing and swelling capacity. Hydrogels with polymers according to the first aspect showed uniform conductivity, low electrical impedance, cohesive strength, appropriate skin adhesion, and biocompatible and capable of repeated use and have been used to electrocardiograph (ECG) electrodes, defibrillation electrode, electrosurgical grounding pads, and iontophoretic drug delivery electrodes. Polymers according to the first aspect can be used as the absorbing hydrogel and the tackifier component of wound dressings.

In at least one embodiment, the composition comprising a polymer according to the first aspect relates to a life sciences composition. The polymers according to the first aspect can be used in pharmaceuticals and in pharmaceutical compositions, in medical manufacturing and in medical devices.

In at least one embodiment, the composition comprising a polymer according to the first aspect relates to hygiene compositions. The polymers according to the first aspect demonstrate a high absorbing and swelling capacity of organic solvents like ethanol, ethyl acetate, or dimethyl ketone. Hydrogels of organic solvents are used in disinfection

products.

In at least one embodiment, the composition comprising a polymer according to the first aspect relates to oil field compositions. Polymers according to the first aspect in oil field compositions have to stand hostile environments and require thermal and hydrolytic stability and the resistance to hard water containing metal ions. For example, in drilling and cementing operations where conditions of high salinity, high temperature, and high pressure are present, these polymer can inhibit fluid loss and be used in oil field environments as scale inhibitors, pipeline flow improvers, as additives in refinery process chemicals, friction reducers and water-control polymers, and in polymer flooding compositions.

In at least one embodiment, the composition comprising a polymer according to the first aspect relates to water treatment compositions. The cation stability of the ACDMT containing polymers are very useful for water treatment processes. Such polymers with low molecular weights cannot only inhibit calcium, magnesium, and silica scale in cooling towers and boilers, but also help corrosion control by dispersing iron oxide. When high molecular weight polymers are used, they can be used to precipitate solids in the treatment of industrial effluent stream.

In at least one embodiment, the composition comprising a polymer according to the first aspect relates to crop protection compositions. Polymers according to the first aspect increase, in dissolved and nanoparticulate polymer formulations, the bioavailability of pesticides in aqueous-organic formulations.

In at least one embodiment, the composition comprising a polymer according to the first aspect relates to membranes. Polymers according to the first aspect increase water flow, retention and fouling resistance of asymmetric ultrafiltration and microfiltration membranes and is being studied as an anionic component in polymer fuel cell membranes.

In at least one embodiment, the composition comprising a polymer according to the first aspect relates to construction compositions. Polymers according to the first aspect can be use as superplasticizers to reduce water in concrete formulations. Benefits of these additives include improved strength, improved workability, improve durability of cement mixtures. Dispersible polymer powder, when bio based ACDMT is introduced, in cement mixtures control air pore content and prevent agglomeration of powders during the spray-drying process from the powder manufacturing and storage. Coating compositions with polymers according to the first aspect prevent calcium ions from being formed as lime on concrete surface and improve the appearance and durability of coating.

In at least one embodiment, the composition comprising a polymer according to the first aspect relates to a fire-fighting device composition for the firegrade A. The liquid polymer - solution absorbs a multiple amount of its own weight in water and forms an adhesive and heat-shielding gel which contains no air bubbles but consists of evenly thickened water. The composition comprising a polymer according to the first aspect has a very good adhesive quality. It even sticks in thickness up to 10 mm at smooth, vertical surfaces (i.e. windows) or on ceilings.

In at least one embodiment, the composition comprising a polymer according to the first aspect relates to lubricant and fuel compositions.

In at least one embodiment, the composition comprising a polymer according to the first aspect relates to driveline compositions.

In at least one embodiment, the composition comprising a polymer according to the first aspect relates to engine oil compositions.

In at least one embodiment, the composition comprising a polymer according to the first aspect relates to fuel compositions.

In at least one embodiment, the composition comprising a polymer according to the first aspect relates to industrial lubricant compositions.

In at least one embodiment, the composition comprising a polymer according to the first aspect can use for ion exchange resins.

EXAMPLES

The examples which follow are intended to illustrate the subject matter of the invention, without restricting it thereto.

Polymerization process A: General precipitation polymerization procedure in tert.-butanol

Dose in a 1-Liter Quickfit round bottom flask equipped with a reflux condenser, sub surface gas inlet tubing, inner temperature sensor and overhead agitator 400 g tert.-butanol with a water content of 3 wt.-%. Charge 69 g bio-based ACDMT according to the invention and 9.2 g carboxyethyl acrylate. Neutralize the ACDMT to a pH of 7 to 8 by injection of gaseous ammonia above the surface. Keep the temperature below 40 °C. Dose 0.93 g GPTA as a crosslinker and 3.71 g methyl acrylate as a neutral monomer according to table A. Inject nitrogen subsurface for 1 h at agitation of 200 rpm. During this time the temperature of the reaction mixture is raised and stabilized to 60 °C with help of a water bath. Readjust the pH at 60 °C to a pH of 7 to 8. The reaction is initiated by the dosage of radical building compound, 1.1 g V-601.

After a few minutes the start of polymerization becomes obvious due to the rising temperature and the precipitation of a polymer. When the temperature maximum is reached, heat the reaction to a gentle reflux for two hours. Cool the reaction mixture to room temperature and dry the polymer suspension at 60 °C under a vacuum of 150 mbar.

Polymerization process B: General precipitation polymerization procedure in tert.-butanol / dimethylketone mixture

Dose in a 1-Liter Quickfit round bottom flask equipped with a reflux condenser, sub surface gas inlet tubing, inner temperature sensor and overhead agitator 200 g tert.-butanol and 200 g dimethylketone with a water content of 2.5 wt.-%. Charge 90 g bio-based ACDMT according to the invention and 8.25 g carboxyethyl acrylate. Neutralize the ACDMT and the carboxyethyl acrylate by charging 41.3 g sodium hydrogen carbonate. Keep the temperature below 40 °C. Dose 0.88 g GPTA, as a crosslinker and a 0.44 g methyl acrylate as a neutral monomer according to table B. Inject nitrogen subsurface for 1 h at agitation of 200 rpm. During this time the temperature of the reaction mixture is raised and stabilized to 60 °C with help of a water bath. Readjust the pH at 60 °C to a pH of 7 to 8. The reaction is initiated by the dosage of radical building compound, 1.1 g V-601

After a few minutes the start of polymerization becomes obvious due to the rising temperature and the precipitation of a polymer. When the temperature maximum is reached, heat the reaction to a gentle reflux for two hours. Cool the reaction mixture to room temperature and dry the polymer suspension at 60 °C under a vacuum of 150 mbar.

Table a): Polymers according to polymerization process A:

| Name | ACDMT / mol-% | Anionic monomer | | Neutral Monomer | | Optional Unit | | Crosslinker | | Initiator | |
|---|---|---|---|---|---|---|---|---|---|---|---|
| | | Name | / mol-% | Name | / mol-% | Name | / mol-% | Name | / mol-% | Name | / g |
| Polymer-A 1 | 75.3 | Carboxyethy acrylate | 14.5 | Methyl acrylate | 9.7 | - | - | GPTA | 0.49 | V-601 | 1.10 |
| Polymer-A 2 | 66.8 | Carboxyethy acrylate | 18.0 | DMAAm | 14.7 | - | - | PEAS | 0.50 | DLP | 1.60 |
| Polymer-A 3 | 88.0 | Carboxyethy acrylate | 11.59 | Methyl acrylate | 0.01 | - | - | GPTA | 0.40 | V-601 | 1.10 |
| Polymer-A 4 | 83.3 | Carboxyethy acrylate oligo | 10.5 | Stearylpoly-ethoxy-(8)-methacrylate | 6.2 | - | - | TMPTA | 0.01 | DLP | 1.80 |
| Polymer-A 5 | 89.4 | Carboxyethy acrylate oligo | 10.0 | Methyl acrylate | 0.1 | - | - | TMPMTA | 0.50 | V-601 | 1.10 |
| Polymer-A 6 | 88.9 | Carboxyethy acrylate oligo | 9.9 | Methyl acrylate | 0.1 | - | - | PEG 600 DMA | 1.01 | V-601 | 1.10 |
| Polymer-A 7 | 76.2 | Methacrylic acid | 2.6 | DMAAm | 20.7 | - | - | GPTA | 0.52 | V-601 | 1.40 |
| Polymer-A 8 | 74.0 | Methacrylic acid | 5.0 | DMAAm | 20.1 | - | - | GPTA | 0.85 | V-601 | 1.50 |
| Polymer-A 9 | 73.8 | Methacrylic acid | 5.0 | DMAAm | 20.1 | - | - | PEAS | 1.18 | V-601 | 1.50 |
| Polymer-A 10 | 90.5 | Methacrylic acid | 5.4 | Behenylpoly-ethoxy-(25)-methacrylate | 3.3 | - | - | TMPTA | 0.75 | DLP | 1.75 |
| Polymer-A 11 | 84.6 | Methacrylic acid | 9.0 | Stearylpoly-ethoxy-(8)-methacrylate | 6.3 | - | - | TMPTA | 0.01 | DLP | 1.80 |
| Polymer-A 12 | 74.0 | Methacrylic acid | 5.0 | DMAAm | 20.1 | - | - | GPTA | 0.85 | V-601 | 1.50 |
| Polymer-A 13 | 90.5 | Methacrylic acid | 5.4 | Behenylpoly-ethoxy-(25)-methacrylate | 3.3 | - | - | TMPTA | 0.75 | DLP | 1.75 |
| Polymer-A 14 | 86.0 | Acrylic acid | 6.0 | Laurylpoly-ethoxy-(7)-methacrylate | 8.0 | - | - | PEAS | 0.01 | DLP | 1.80 |
| Polymer-A 15 | 93.4 | Acrylic acid | 6.0 | Methyl acrylate | 0.1 | - | - | GPTA | 0.45 | V-601 | 1.10 |

(continued)

| Name | ACDMT / mol-% | Anionic monomer | | Neutral Monomer | | Optional Unit | | Crosslinker | | Initiator | |
|---|---|---|---|---|---|---|---|---|---|---|---|
| | | Name | / mol-% | Name | / mol-% | Name | / mol-% | Name | / mol-% | Name | / g |
| Polymer-A 16 | 93.1 | Acrylic acid | 6.0 | Methyl acrylate | 0.5 | - | - | GPTA | 0.45 | V-601 | 1.10 |
| Polymer-A 17 | 87.6 | 2-ethylacrylic acid | 11.5 | Methyl acrylate | 0.5 | - | - | PEAS | 0.42 | V-601 | 1.10 |
| Polymer-A 18 | 94.4 | Itaconic acid | 5.0 | Methyl acrylate | 0.1 | - | - | GPTA | 0.46 | V-601 | 1.10 |
| Polymer-A 19 | 91.0 | Itaconic acid | 1.0 | Laurylpoly-ethoxy-(7)-methacrylate | 8.0 | - | - | TMPTA | 0.01 | DLP | 1.80 |
| Polymer-A 20 | 94.0 | Itaconic acid | 5.0 | Methyl acrylate | 0.5 | - | - | GPTA | 0.46 | V-601 | 1.10 |
| Polymer-A 21 | 80 | Carboxyethyl acrylate | 5 | DMAAm | 4.5 | NVP | 5 | GPTA | 0.5 | V-601 | 1.10 |
| Polymer-A 22 | 74.25 | Methacrylic acid | 5 | Laurylpoly-ethoxy-(7)-methacrylate | 10 | DMAAm | 10 | TMPTA | 0.75 | DLP | 1.80 |
| Polymer-A 23 | 85 | Acrylic acid | 5 | DMAAm | 4.5 | NVP | 5 | PEAS | 0.5 | V-601 | 1.10 |
| Polymer-A 24 | 89 | 2-ethylacrylic acid | 5 | DMAAm | 2.5 | Methyl acrylate | 2.5 | TMPMTA | 1 | V-601 | 1.10 |
| Polymer-A 25 | 88.5 | Itaconic acid | 5 | DMAAm | 1.5 | NVP | 3.5 | PEG 600 DMA | 1.5 | V-601 | 1.10 |

ACDMT = acryloyldimethyltaurate, NVP = N-vinylpyrollidone, DMAAm = dimethylacrylamide, TMPTA = trimethylolpropane triacrylate, TMPTA = trimethylolpropane triacrylate, TMPTMA = trimethylolpropane trimethacrylate, GPTA = glycerinpropoxylate triacrylate, PEAS = pentaerythritoldiacrylate monostearate, DLP = dilaurylperoxide, V-601 = dimethyl 2,2'-azobis(2-methylpropionate), PEG 600 DMA = polyethylene glycol dimethacrylate (600 g/mol).

Table B): Polymers according to polymerization process B:

| Name | ACDMT | Anionic monomer | | NaHCOs | Neutral Monomer | | Optional Unit | | Crosslinker | | Initiator | |
|---|---|---|---|---|---|---|---|---|---|---|---|---|
| | / mol-% | Name | / mol-% | Name | Name | / mol-% | Name | / mol-% | Name | / mol-% | Name | / g |
| Polymer-B 1 | 87.1 | Carboxy ethylacrylate | 11.5 | 41.3 | Methyl acrylate | 1.0 | - | - | GPTA | 0.41 | V-601 | 1.10 |
| Polymer-B 2 | 83.3 | Carboxy ethylacrylate | 10.5 | 36.5 | Stearylpoly-ethoxy-(8)-methacrylate | 6.2 | - | - | TMPTA | 0.01 | DLP | 1.80 |
| Polymer-B 3 | 83.0 | Carboxy ethylacrylate | 9.0 | 35.9 | Laurylpoly-ethoxy-(7)-methacrylate | 8.0 | - | - | PEAS | 0.01 | DLP | 1.80 |
| Polymer-B 4 | 88.0 | Carboxy ethylacrylate | 11.59 | 41.3 | Methyl acrylate | 0.01 | - | - | GPTA | 0.40 | V-601 | 1.10 |
| Polymer-B 5 | 88.5 | Carboxy ethylacrylate oligo | 9.9 | 40.5 | Methyl acrylate | 0.1 | - | - | GPTA | 1.50 | V-601 | 1.10 |
| Polymer-B 6 | 67.9 | Carboxy ethylacrylate oligo | 8.5 | 36.5 | DMAAm | 23.1 | - | - | GPTA | 0.50 | DLP | 1.30 |
| Polymer-B 7 | 79.0 | Methacrylic acid | 10.2 | 50.3 | DMAAm | 10.1 | - | - | PEAS | 0.70 | V-601 | 1.50 |
| Polymer-B 8 | 74.0 | Methacrylic acid | 5.0 | 43.3 | DMAAm | 20.1 | - | - | GPTA | 0.85 | V-601 | 1.50 |
| Polymer-B 9 | 90.9 | Methacrylic acid | 8.5 | 39.9 | Methyl acrylate | 0.1 | - | - | GPTA | 0.44 | V-601 | 1.10 |
| Polymer-B 10 | 89.0 | Methacrylic acid | 3.0 | 33.5 | Laurylpoly-ethoxy-(7)-methacrylate | 8.0 | - | - | TMPTA | 0.01 | DLP | 1.80 |
| Polymer-B 11 | 74.0 | Methacrylic acid | 5.0 | 43.3 | DMAAm | 20.1 | - | - | GPTA | 0.9 | V-601 | 1.50 |
| Polymer-B 12 | 90.6 | Methacrylic acid | 8.5 | 39.9 | Methyl acrylate | 0.5 | - | - | GPTA | 0.44 | V-601 | 1.10 |
| Polymer-B 13 | 89.7 | Acrylic acid | 6.0 | 34.6 | Behenylpoly-ethoxy-(25)-methacrylate | 3.3 | - | - | TMPTA | 1.02 | DLP | 1.75 |

(continued)

| Name | ACDMT | Anionic monomer | | NaHCOs | Neutral Monomer | | Optional Unit | | Crosslinker | | Initiator | |
|---|---|---|---|---|---|---|---|---|---|---|---|---|
| | / mol-% | Name | / mol-% | Name | Name | / mol-% | Name | / mol-% | Name | / mol-% | Name | / g |
| Polymer-B 14 | 87.5 | Acrylic acid | 6.0 | 34.6 | Stearylpoly-ethoxy-(8)-methacrylate | 6.5 | - | - | PEAS | 0.01 | DLP | 1.80 |
| Polymer-B 15 | 93.0 | Acrylic acid | 3.0 | 33.5 | Behenylpoly-ethoxy-(25)-methacrylate | 3.3 | - | - | TMPTA | 0.65 | DLP | 1.75 |
| Polymer-B 16 | 90.4 | 2-propylacrylic acid | 9.0 | 40.1 | Methyl acrylate | 0.1 | - | - | GPTA | 0.44 | V-601 | 1.10 |
| Polymer-B 17 | 90 | Carboxy ethylacrylate | 2 | 35.9 | DMMAA | 4.5 | NVP | 3 | GPTA | 0.5 | DLP | 1.75 |
| Polymer-B 18 | 85 | Carboxy ethylacrylate oligo | 7 | 35.9 | Stearylpoly-ethoxy-(8)-methacrylate | 3 | NVP | 4 | TMPTA | 1 | V-601 | 1.10 |
| Polymer-B 19 | 86.8 | Acrylic acid | 7 | 36.5 | Laurylpoly-ethoxy-(7)-methacrylate | 3 | NVP | 2.7 | TMPTA | 0.5 | DLP | 1.75 |
| Polymer-B 20 | 86.8 | Methacrylic acid | 7 | 36.5 | Behenylpoly-ethoxy-(25)-methacrylate | 3.3 | NVP | 2.2 | GPTA | 0.7 | V-601 | 1.10 |

ACDMT = acryloyldimethyltaurate, NVP = N-vinylpyrollidone, DMAAm = dimethylacrylamide, TMPTA = trimethylolpropane triacrylate, TMPTA = trimethylolpropane triacrylate, TMPTMA = trimethylolpropane trimethacrylate, GPTA = glycerinpropoxylate triacrylate, PEAS = pentaerythritoldiacrylate monostearate, DLP = dilaurylperoxide, V-601 = dimethyl 2,2'-azobis(2-methylpropionate).

Analytical methods

Determination of the Fickenscher k-value:

This method was used to determine the k-value of certain polymers according to DIN EN ISO 1628-1.
A k-value measurement was a way to indirectly analyze the molecular weight/size of a polymer. A comparatively higher K-value corresponds to a larger molecular weight/size as compared to a polymer with the same composition and made by the same process.
By measuring the measuring the pass-through time of a solvent ($t^0$) and the pass-through time of a polymer solution (tc) through the capillary of an Ubbelhode viscometer the relative viscosity was determined.

$$Z = \frac{t_c}{t_0} = \frac{\eta_c}{\eta_0}$$

From the relative viscosity z the k-value can be calculated according to

$$\lg z = \left[\frac{75k^2}{1+150k \times c} + k\right] \times 1$$

In this case

$$k = \frac{1{,}5\lg z - 1 \pm \sqrt{1 + \left(\frac{2}{c} + 2 + 1{,}5\lg z\right) 1{,}5\lg z}}{150 + 300c}$$

k-value =1000 k
Here in it was defined:

$$Z = \frac{t_c}{t_0} = \frac{\eta_c}{\eta_0} \quad \text{relative Viscosity,}$$

$\eta_c$ dynamic viscosity of the solution,

$\eta_o$ dynamic viscosity of the solvent and

c mass concentration of polymer in solution in in g/cm$^3$

Alternatively the k-value can be evaluated from lists provided by the manufacturer of the equipment.
After determination of the mass concentration of the polymer solution by microwave drying with a CEM Smart 5 at 120 °C, 20 ml of a 0.5 % polymer solution was prepared. 16 to 18 ml of the solution was measured in an Ubbelhode capillary viscometer at 25 °C. The Ubbelhode viscometer was chose to have a pass-through time of 100 to 120 s. It was measured in a Schott AVS viscometer, combined with a CT 1150 Thermostate and flow cooler CK 100.
The IT unit calculated the k-value.

Brookfield viscosity in 1% solution:

Brookfield viscosity was determined with a Brookfield viscometer model LV, RVT DV-II or LVT DV-II.
In a 600 ml beaker, 4 g dry Polymer was dissolved in 394 g distilled water. The solution was stirred for 2 h at 20 °C with a finger stirrer driven by an overhead agitator at 200 rpm. Then the polymer solution, free of entrapped air, was tempered for 16 h at 20 °C. The spindle was chosen to measure between 20 to 80 % of the scale at 20 rpm.
Brookfield viscosity in solution as is.
Brookfield viscosity was determined with a Brookfield viscometer model LV, RVT DV-II or LVT DV-II.

In a 600 ml beaker, the polymer solution, free of entrapped air, was tempered for 2 h at 20 °C. The spindle was chosen to measure between 20 to 80 % of the scale at 20 rpm.

Analytical procedure for determination of bio-based content according to ASTM 6866-12, Method B:
The provided sample material did not undergo any pre-treatment procedure and was converted to graphite as was using the following procedure.

Depending on the estimated amount of carbon content, typically a few milligram of sample material was being combusted in an Elemental Analyzer (EA). The resulting gas mixture was being cleaned and $CO_2$ was automatically separated by the EA using the purge and trap technology.

The remaining $CO_2$ was transferred into a custom-made graphitization system, converted into carbon (graphite) catalytically using $H_2$ and an iron-powder catalyst.

The carbon-14 determination of the graphite was performed at the Klaus-Tschira-Archaeomtrie-Center using an accelerator mass-spectrometer (AMS) of the type MICADAS (developed at the ETH Zurich, Switzerland).

## Claims

**1.** A polymer comprising:

(a) from 9.49 mol-% to 98 mol-%, preferably from 27.5 mol-% to 97.4 mol-% repeating units (a) resulting from the incorporation of acryloyldimethyltaurate wherein at least 10 wt.-%, preferably at least 20 wt.- % of the repeating units resulting from the incorporation of acryloyldimethyltaurate comprise from 28 wt.-% to 100 wt.-% bio-based carbon content, relative to the total mass of carbon in the repeating unit (a) resulting from the incorporation of acryloyldimethyltaurate, measured according to standard ASTM D6866-12, Method B;

wherein the acryloyldimethyltaurate is prepared from acrylonitrile, isobutene and a mixture of sulfuric acid and fuming sulfuric acid comprising sulfur trioxide, wherein at least one of acrylonitrile and isobutene is of bio-based origin, and
wherein the sulfonic acid groups of the repeating units resulting from the incorporation of acryloyldimethyltaurate comprise counterions $Q^+$,
wherein $Q^+$ is $H^+$, $NH_4^+$, organic ammonium ions $[NHR^5R^6R^7]^+$ wherein $R^5$, $R^6$, and $R^7$ independently of one another may be hydrogen, a linear or branched alkyl group having 1 to 22 carbon atoms, a linear or branched, singularly or multiply unsaturated alkenyl group having 2 to 22 carbon atoms, a $C_6$-$C_{22}$ alkylamidopropyl group, a linear mono-hydroxyalkyl group having 2 to carbon atoms or a linear or branched dihydroxyalkyl group having 3 to carbon atoms, and where at least one of the radicals $R^5$, $R^6$, and $R^7$ is not hydrogen, or $Q^+$ is $Li^+$, $Na^+$, $K^+$, ½ $Ca^{++}$, ½ $Mg^{++}$, ½ $Zn^{++}$, 1/3 $Al^{+++}$, or combinations thereof;

(b) from 0.01 mol-% to 5 mol-%, preferably from 0.01 mol-% to 4 mol-% crosslinking or branching units, wherein the crosslinking or branching units result from the incorporation of a monomer comprising at least two olefinically unsaturated double bonds;
(c) from 0.01 mol-% to 88.52 mol-%, preferably from 0.05 mol-% to 72.4 mol-% of repeating neutral structural units, preferably wherein at least 10 wt.-%, preferably at least 20 wt.-% of the repeating neutral structural units comprise from 0 wt.-% to 100 wt.-% bio-based carbon content, relative to the total mass of carbon in the repeating neutral structural unit, measured according to standard ASTM D6866-12, Method B;
(d) from 1.98 mol-% to 20 mol-%, preferably from 2.5 mol-% to 18 mol-% of repeating anionic structural units, wherein the repeating anionic structural units result from the incorporation of a monomer comprising at least one carboxylate anion, and wherein the repeating anionic structural units are different from units (a), preferably wherein at least 10 wt.-%, preferably at least 20 wt.-% of the repeating anionic structural units comprise from 0 wt.-% to 100 wt.-% bio-based carbon content, relative to the total mass of carbon in the repeating anionic structural unit, measured according to standard ASTM D6866-12, Method B.

**2.** The polymer according to claim 1, wherein at least 25 wt.-%, or at least 30 wt.-%, or at least 35 wt.-%, or at least 40 wt.-%, or at least 45 wt.-%, or at least 50 wt.-%, or at least 55 wt.-%, or at least 60 wt.-%, or at least 65 wt.-%, or at least 70 wt.-%, or at least 75 wt.-%, or at least 80 wt.-%, or at least 85 wt.-%, or at least 90 wt.-%, or at least 95 wt.-%, or at least 96 wt.-%, or at least 97 wt.-%, or at least 98 wt.-%, or at least 99 wt.-%, or at least 99.5 wt.-% of the repeating units (a) resulting from the incorporation of acryloyldimethyltaurate comprise from 28 wt.-% to 100 wt.-% bio-based carbon content, relative to the total mass of carbon in the repeating unit resulting from the incorporation of acryloyldimethyltaurate, measured according to standard ASTM D6866-12, Method B.

**3.** The polymer according to any of claims 1 or 2, wherein the repeating units (a) resulting from the incorporation of acryloyldimethyltaurate have a degree of neutralisation of from 50.0 to 100 mol-%, preferably from 80 mol-% to 100 mol- %, more preferably from 90.0 to 100 mol-%, even more preferably from 95.0 to 100 mol-%.

**4.** The polymer according to any of claims 1 to 3, wherein $Q^+$ is $H^+$, $Li^+$, $Na^+$, $K^+$, ½ $Ca^{++}$, ½ $Mg^{++}$, ½ $Zn^{++}$, 1/3 $Al^{+++}$, or combinations thereof.

**5.** The polymer according to any of claims 1 to 3, wherein $Q^+$ is $Na^+$.

**6.** The polymer according to any of claims 1 to 5, wherein the polymer comprises from 37 mol-% to 96.4 mol-%, preferably from 43 mol-% to 95.3 mol-% units (a), from 0.1 mol-% to 3 mol-%, preferably from 0.2 mol-% to 2 mol-% units (b), from 0.1 mol-% to 59.3 mol-%, preferably from 0.5 mol-% to 52.8 mol-% units (c), and from 3.5 mol-% to 16 mol-%, preferably from 4 mol-% to 14 mol-% units (d).

**7.** The polymer according to any of claims 1 to 6, wherein the acryloyldimethyltaurate comprises from 35 wt.-%, preferably from 40 wt.-%, more preferably from 54 wt.-%, even more preferably from 57 wt.-% to 100 wt.-% bio-based carbon content, relative to the total mass of carbon in the acryloyldimethyltaurate, measured according to standard ASTM D6866-12, Method B.

**8.** The polymer according to any of claims 1 to 7, wherein the acryloyldimethyltaurate comprises 100 wt.-% bio-based carbon content, relative to the total mass of carbon in the acryloyldimethyltaurate, measured according to standard ASTM D6866-12, Method B.

**9.** The polymer according to any of claims 1 to 8, wherein the crosslinking or branching units result from the incorporation of a monomer according to Formula (4)

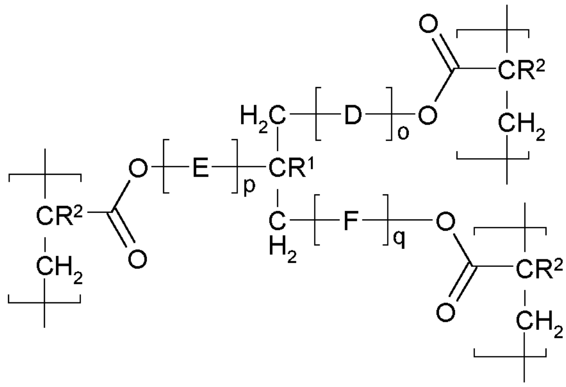

(4)

wherein

$R^1$ is independently selected from H, methyl or ethyl; and
$R^2$ is a linear or branched alkyl group having 1 to 6 carbon atoms, or is a linear or branched, mono- or polyunsaturated alkylene group having 2 to 6 carbon atoms;
D, E, and F are independently methyleneoxy($-CH_2O$), ethyleneoxy($-CH_2-CH_2-O-$), propyleneoxy($-CH(CH_3)-CH_2-O-$), a linear or branched alkylene group having 1 to 6 carbon atoms, a linear or branched, singularly or multiply unsaturated alkenylene group having 2 to 6 carbon atoms, a linear mono-hydroxyalkylene group having 2 to 6 carbon atoms or a linear or branched dihydroxyalkylene group having 3 to 6 carbon atoms; and
o, p, and q each independently are an integer from 1 to 50.

**10.** The polymer according to any of claims 1 to 9, wherein the crosslinking or branching units (b) result from the incorporation of a crosslinker selected from the group consisting of trimethylolpropane triacrylate (TMPTA) and/or glycerol propoxylate triacrylate (GPTA).

**11.** The polymer according to any of claims 1 to 10, wherein the polymer consists of units (a), units (b), units (c) and units (d).

**12.** The polymer according to any of claims 1 to 11, wherein the polymer has a weight average molecular weight of at least 700 g/mol, preferably from 700 g/mol to 10 million g/mol.

**13.** A process for obtaining a polymer by polymerization of: (a) at least one acryloyldimethyltaurate monomer according Formula (10) comprising from 28 wt.- % to 100 wt.-% bio-based carbon content, relative to the total mass of carbon in the acryloyldimethyltaurate monomer according to Formula (10), measured according to standard ASTM D6866-12, Method B; (b) at least one crosslinking or branching monomer; (c) at least one neutral monomer; and (d) at least one anionic monomer;

wherein the crosslinking or branching monomer has at least two olefinically unsaturated double bonds; and wherein Formula (10) is:

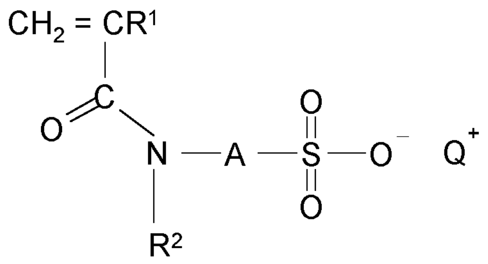

(10)

wherein: $R^1$ and $R^2$ are H; A is $-C(CH_3)_2-H_2C-$; and
wherein the acryloyldimethyltaurate monomer is prepared from acrylonitrile, isobutene and a mixture of sulfuric acid and fuming sulfuric acid comprising sulfur trioxide, wherein at least one of acrylonitrile and isobutene is of bio-based origin; and
wherein $Q^+$ is $H^+$, $NH_4^+$, organic ammonium ions $[NHR^5R^6R^7]^+$ wherein $R^5$, $R^6$, and $R^7$ independently of one another may be hydrogen, a linear or branched alkyl group having 1 to 22 carbon atoms, a linear or branched, singularly or multiply unsaturated alkenyl group having 2 to 22 carbon atoms, a $C_6$-$C_{22}$ alkylamidopropyl group, a linear mono-hydroxyalkyl group having 2 to carbon atoms or a linear or branched dihydroxyalkyl group having 3 to carbon atoms, and where at least one of the radicals $R^5$, $R^6$, and $R^7$ is not hydrogen, or $Q^+$ is $Li^+$, $Na^+$, $K^+$, ½ $Ca^{++}$, ½ $Mg^{++}$, ½ $Zn^{++}$, 1/3 $Al^{+++}$, or combinations thereof.

**14.** Use of the polymer according to any of claims 1 to 12 as a as a thickening agent and/or rheology modifier and/or a viscosity modifier.

**15.** A composition comprising:

(a) A polymer according to any of claims 1 to 12; and
(b) A further component.

**Patentansprüche**

**1.** Polymer, umfassend:

(a) 9,49 Mol-% bis 98 Mol-%, vorzugsweise von 27,5 Mol-% bis 97,4 Mol-% wiederkehrende Einheiten (a), die sich aus dem Einbau von Acryloyldimethyltaurat ergeben, wobei mindestens 10 Gew.-%, vorzugsweise mindestens 20 Gew.-%, der wiederkehrenden Einheiten, die sich aus dem Einbau von Acryloyldimethyltaurat ergeben, 28 Gew.-% bis 100 Gew.-% Gehalt an biobasiertem Kohlenstoff, bezogen auf die Gesamtmasse von Kohlenstoff in der wiederkehrenden Einheit (a), die sich aus dem Einbau von Acryloyldimethyltaurat ergibt, umfassen, gemessen gemäß ASTM-Norm D6866-12, Methode B;

wobei das Acryloyldimethyltaurat aus Acrylnitril, Isobuten und einer Mischung von Schwefelsäure und rauchender Schwefelsäure, die Schwefeltrioxid umfasst, hergestellt wird,
wobei das Acrylnitril und/oder das Isobuten biobasierter Herkunft sind, und
wobei die Sulfonsäuregruppen der wiederkehrenden Einheiten, die sich aus dem Einbau von Acryloyldimethyltaurat ergeben, Gegenionen $Q^+$ umfassen, wobei $Q^+$ für $H^+$, $NH_4^+$, organische Ammoniumionen

$[NHR^5R^6R^7]^+$, wobei $R^5$, $R^6$ und $R^7$ unabhängig voneinander für Wasserstoff, eine lineare oder verzweigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine $C_6$-$C_{22}$-Alkylamidopropylgruppe, eine lineare Monohydroxyalkylgruppe mit 2 bis Kohlenstoffatomen oder eine lineare oder verzweigte Dihydroxyalkylgruppe mit 3 bis Kohlenstoffatomen stehen können und wobei mindestens einer der Reste $R^5$, $R^6$ und $R^7$ nicht für Wasserstoff steht, oder $Q^+$ für $Li^+$, $Na^+$, $K^+$, ½ $Ca^{++}$, ½ $Mg^{++}$, ½ $Zn^{++}$, 1/3 $Al^{+++}$ oder Kombinationen davon steht;

(b) 0,01 Mol-% bis 5 Mol-%, vorzugsweise 0,01 Mol- % bis 4 Mol-%, Vernetzungs- oder Verzweigungseinheiten, wobei sich die Vernetzungs- oder Verzweigungseinheiten aus dem Einbau eines Monomers mit mindestens zwei olefinisch ungesättigten Doppelbindungen ergeben;
(c) 0,01 Mol-% bis 88,52 Mol-%, vorzugsweise 0,05 Mol-% bis 72,4 Mol-%, wiederkehrende neutrale Struktureinheiten, vorzugsweise wobei mindestens 10 Gew.-%, vorzugsweise mindestens 20 Gew.-%, der wiederkehrenden neutralen Struktureinheiten 0 Gew.-% bis 100 Gew.-% Gehalt an biobasiertem Kohlenstoff, bezogen auf die Gesamtmasse von Kohlenstoff in der wiederkehrenden neutralen Struktureinheit, umfassen, gemessen gemäß ASTM-Norm D6866-12, Methode B;
(d) 1,98 Mol-% bis 20 Mol-%, vorzugsweise 2,5 Mol- % bis 18 Mol-%, wiederkehrende anionische Struktureinheiten, wobei sich die wiederkehrenden anionischen Struktureinheiten aus dem Einbau eines Monomers mit mindestens einem Carboxylatanion ergeben und wobei die sich wiederholenden anionischen Struktureinheiten von Einheiten (a) verschieden sind, vorzugsweise wobei mindestens 10 Gew.-%, vorzugsweise mindestens 20 Gew.-%, der wiederkehrenden anionischen Struktureinheiten 0 Gew.-% bis 100 Gew.-% Gehalt an biobasiertem Kohlenstoff, bezogen auf die Gesamtmasse von Kohlenstoff in der wiederkehrenden anionischen Struktureinheit, umfassen, gemessen gemäß ASTM-Norm D6866-12, Methode B.

**2.** Polymer nach Anspruch 1, wobei mindestens 25 Gew.-% oder mindestens 30 Gew.-% oder mindestens 35 Gew.-% oder mindestens 40 Gew.-% oder mindestens 45 Gew.-% oder mindestens 50 Gew.-% oder mindestens 55 Gew.-% oder mindestens 60 Gew.-% oder mindestens 65 Gew.-% oder mindestens 70 Gew.-% oder mindestens 75 Gew.-% oder mindestens 80 Gew.-% oder mindestens 85 Gew.-% oder mindestens 90 Gew.-% oder mindestens 95 Gew.-% oder mindestens 96 Gew.-% oder mindestens 97 Gew.-% oder mindestens 98 Gew.-% oder mindestens 99 Gew.-% oder mindestens 99,5 Gew.-% der wiederkehrenden Einheiten (a), die sich aus dem Einbau von Acryloyldimethyltaurat ergeben, 28 Gew.-% bis 100 Gew.-% Gehalt an biobasiertem Kohlenstoff, bezogen auf die Gesamtmasse von Kohlenstoff in der wiederkehrenden Einheit, die sich aus dem Einbau von Acryloyldimethyltaurat ergibt, umfassen, gemessen gemäß ASTM-Norm D6866-12, Methode B.

**3.** Polymer nach Anspruch 1 oder 2, wobei die wiederkehrenden Einheiten (a), die sich aus dem Einbau von Acryloyldimethyltaurat ergeben, einen Neutralisationsgrad von 50,0 bis 100 Mol-%, vorzugsweise von 80 Mol-% bis 100 Mol-%, weiter bevorzugt von 90,0 bis 100 Mol-%, noch weiter bevorzugt von 95,0 bis 100 Mol-%, aufweisen.

**4.** Polymer nach einem der Ansprüche 1 bis 3, wobei $Q^+$ für $H^+$, $Li^+$, $Na^+$, $K^+$, ½ $Ca^{++}$, ½ $Mg^{++}$, ½ $Zn^{++}$, 1/3 $Al^{+++}$ oder Kombinationen davon steht.

**5.** Polymer nach einem der Ansprüche 1 bis 3, wobei $Q^+$ für $Na^+$ steht.

**6.** Polymer nach einem der Ansprüche 1 bis 5, wobei das Polymer 37 Mol-% bis 96,4 Mol-%, vorzugsweise 43 Mol-% bis 95,3 Mol-%, Einheiten (a), 0,1 Mol-% bis 3 Mol-%, vorzugsweise 0,2 Mol-% bis 2 Mol-%, Einheiten (b), 0,1 Mol-% bis 59,3 Mol-%, vorzugsweise 0,5 Mol-% bis 52,8 Mol-%, Einheiten (c) und 3,5 Mol-% bis 16 Mol-%, vorzugsweise 4 Mol-% bis 14 Mol-%, Einheiten (d) umfasst.

**7.** Polymer nach einem der Ansprüche 1 bis 6, wobei das Acryloyldimethyltaurat 35 Gew.-%, vorzugsweise 40 Gew.-%, weiter bevorzugt 54 Gew.-%, noch weiter bevorzugt 57 Gew.-%, bis 100 Gew.-% Gehalt an biobasiertem Kohlenstoff, bezogen auf die Gesamtmasse von Kohlenstoff in dem Acryloyldimethyltaurat, umfasst, gemessen gemäß ASTM-Norm D6866-12, Methode B.

**8.** Polymer nach einem der Ansprüche 1 bis 7, wobei das Acryloyldimethyltaurat 100 Gew.-% Gehalt an biobasiertem Kohlenstoff, bezogen auf die Gesamtmasse von Kohlenstoff in dem Acryloyldimethyltaurat, umfasst, gemessen gemäß ASTM-Norm D6866-12, Methode B.

**9.** Polymer nach einem der Ansprüche 1 bis 8, wobei sich die Vernetzungs- oder Verzweigungseinheiten aus dem

Einbau eines Monomers gemäß Formel (4) ergeben:

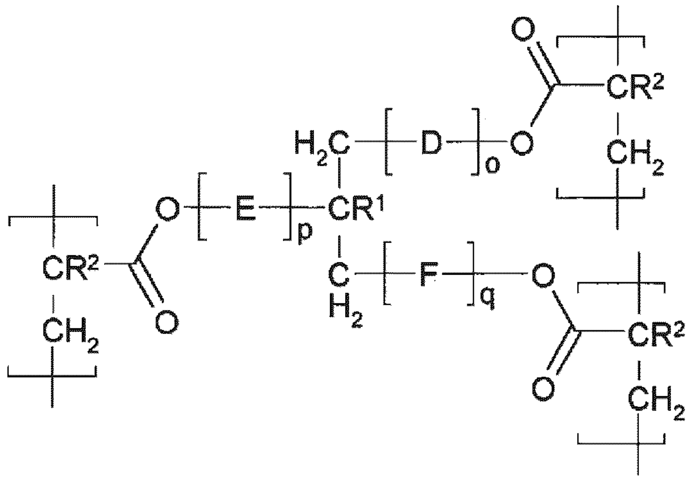

(4)

wobei

$R^1$ unabhängig aus H, Methyl oder Ethyl ausgewählt ist und
$R^2$ für eine lineare oder verzweigte Alkylgruppe mit 1 bis 6 Kohlenstoffatomen oder für eine lineare oder verzweigte, ein- oder mehrfach ungesättigte Alkylengruppe mit 2 bis 6 Kohlenstoffatomen steht;
D, E und F unabhängig für Methylenoxy ($-CH_2O$), Ethylenoxy ($-CH_2-CH_2-O-$), Propylenoxy ($-CH(CH_3)-CH_2-O-$), eine lineare oder verzweigte Alkylengruppe mit 1 bis 6 Kohlenstoffatomen, eine lineare oder verzweigte, ein- oder mehrfach ungesättigte Alkenylengruppe mit 2 bis 6 Kohlenstoffatomen, eine lineare Monohydroxyalkylengruppe mit 2 bis 6 Kohlenstoffatomen oder eine lineare oder verzweigte Dihydroxyalkylengruppe mit 3 bis 6 Kohlenstoffatomen stehen; und
o, p und q jeweils unabhängig voneinander für eine ganze Zahl von 1 bis 50 stehen.

**10.** Polymer nach einem der Ansprüche 1 bis 9, wobei sich die Vernetzungs- oder Verzweigungseinheiten (b) aus dem Einbau eines Vernetzers aus der Gruppe bestehend aus Trimethylolpropantriacrylat (TMPTA) und/oder Glycerinpropoxylattriacrylat (GPTA) ergeben.

**11.** Polymer nach einem der Ansprüche 1 bis 10, wobei das Polymer aus Einheiten (a), Einheiten (b), Einheiten (c) und Einheiten (d) besteht.

**12.** Polymer nach einem der Ansprüche 1 bis 11, wobei das Polymer ein gewichtsmittleres Molekulargewicht von mindestens 700 g/mol, vorzugsweise von 700 g/mol bis 10 Millionen g/mol, aufweist.

**13.** Verfahren zum Erhalt eines Polymers durch Polymerisation von: (a) mindestens einem Acryloyldimethyltaurat-Monomer gemäß Formel (10), umfassend 28 Gew.- % bis 100 Gew.-% Gehalt an biobasiertem Kohlenstoff, bezogen auf die Gesamtmasse von Kohlenstoff in dem Acryloyldimethyltaurat-Monomer gemäß Formel (10), gemessen gemäß ASTM-Norm D6866-12, Methode B; (b) mindestens einem Vernetzungs- oder Verzweigungsmonomer; (c) mindestens einem neutralen Monomer und (d) mindestens einem anionischen Monomer;

wobei das Vernetzungs- oder Verzweigungsmonomer mindestens zwei olefinisch ungesättigte Doppelbindungen aufweist;
und wobei es sich bei Formel (10) um:

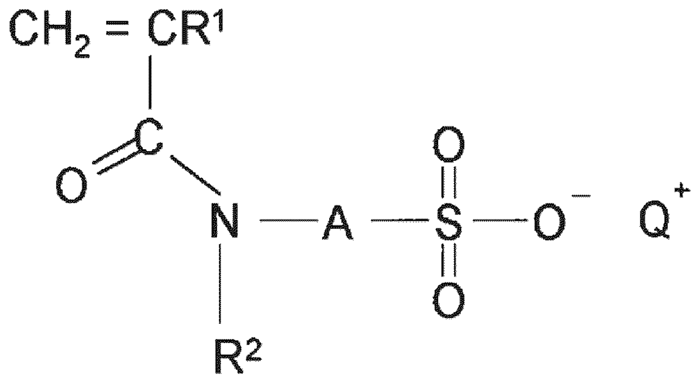

(10)

handelt, wobei $R^1$ und $R^2$ für H stehen; A für $-C(CH_3)_2-H_2C-$ steht; und wobei das Acryloyldimethyltaurat-Monomer aus Acrylnitril, Isobuten und einer Mischung von Schwefelsäure und rauchender Schwefelsäure, die Schwefeltrioxid umfasst, hergestellt wird, wobei das Acrylnitril und/oder das Isobuten biobasierter Herkunft sind; und wobei $Q^+$ für $H^+$, $NH_4^+$, organische Ammoniumionen $[NHR^5R^6R^7]^+$, wobei $R^5$, $R^6$ und $R^7$ unabhängig voneinander für Wasserstoff, eine lineare oder verzweigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine $C_6$-$C_{22}$-Alkylamidopropyl-gruppe, eine lineare Monohydroxyalkylgruppe mit 2 bis Kohlenstoffatomen oder eine lineare oder verzweigte Dihydroxyalkylgruppe mit 3 bis Kohlenstoffatomen stehen können und wobei mindestens einer der Reste $R^5$, $R^6$ und $R^7$ nicht für Wasserstoff steht, oder $Q^+$ für $Li^+$, $Na^+$, $K^+$, ½ $Ca^{++}$, ½ $Mg^{++}$, ½ $Zn^{++}$, 1/3 $Al^{+++}$ oder Kombinationen davon steht.

**14.** Verwendung des Polymers nach einem der Ansprüche 1 bis 12 als Verdickungsmittel und/oder Rheologiemodifikator und/oder Viskositätsmodifikator.

**15.** Zusammensetzung, umfassend:

(a) ein Polymer nach einem der Ansprüche 1 bis 12 und
(b) eine weitere Komponente.

## Revendications

**1.** Polymère comprenant :

(a) de 9,49 % en moles à 98 % en moles, préférablement de 27,5 % en moles à 97,4 % en moles de motifs répétitifs (a) résultant de l'incorporation d'acryloyldiméthyltaurate, au moins 10 % en poids, préférablement au moins 20 % en poids des motifs répétitifs résultant de l'incorporation d'acryloyldiméthyltaurate comprenant une teneur en carbone d'origine biologique de 28 % en poids à 100 % en poids, par rapport à la masse totale de carbone dans le motif répétitif (a) résultant de l'incorporation d'acryloyldiméthyltaurate, mesurée selon la norme ASTM D6866-12, méthode B ;

l'acryloyldiméthyltaurate étant préparé à partir d'acrylonitrile, d'isobutène et d'un mélange d'acide sulfurique et d'acide sulfurique fumant comprenant du trioxyde de soufre, au moins l'un parmi l'acrylonitrile et l'isobutène étant d'origine biologique, et
les groupes acide sulfonique des motifs répétitifs résultant de l'incorporation d'acryloyldiméthyltaurate comprenant des contre-ions $Q^+$, $Q^+$ étant $H^+$, $NH_4^+$, des ions ammonium organiques $[NHR^5R^6R^7]^+$, $R^5$, $R^6$, et $R^7$ indépendamment les uns des autres pouvant être hydrogène, un groupe alkyle linéaire ou ramifié ayant 1 à 22 atomes de carbone, un groupe alcényle linéaire ou ramifié, insaturé une fois ou plusieurs fois, ayant 2 à 22 atomes de carbone, un groupe alkylamidopropyle en $C_{6-22}$, un groupe monohydroxyalkyle linéaire ayant 2 à atomes de carbone ou un groupe dihydroxyalkyle linéaire ou ramifié ayant 3 à atomes de carbone, et au moins l'un des radicaux $R^5$, $R^6$, et $R^7$ n'étant pas hydrogène, ou $Q^+$ étant $Li^+$, $Na^+$, $K^+$, ½ $Ca^{++}$, ½ $Mg^{++}$, ½ $Zn^{++}$, 1/3 $Al^{+++}$, ou des combinaisons correspondantes;

(b) de 0,01 % en moles à 5 % en moles, préférablement de 0,01 % en moles à 4 % en moles de motifs de

réticulation ou de ramification, les motifs de réticulation ou de ramification résultant de l'incorporation d'un monomère comprenant au moins deux doubles liaisons oléfiniquement insaturées ;
(c) de 0,01 % en moles à 88,52 % en moles, préférablement de 0,05 % en moles à 72,4 % en moles de motifs structuraux neutres répétitifs, préférablement au moins 10 % en poids, préférablement au moins 20 % en poids des motifs structuraux neutres répétitifs comprenant une teneur en carbone d'origine biologique de 0 % en poids à 100 % en poids, par rapport à la masse totale de carbone dans le motif structural neutre répétitif, mesurée selon la norme ASTM D6866-12, méthode B ;
(d) de 1,98 % en moles à 20 % en moles, préférablement de 2,5 % en moles à 18 % en moles de motifs structuraux anioniques répétitifs, les motifs structuraux anioniques répétitifs résultant de l'incorporation d'un monomère comprenant au moins un anion carboxylate, et les motifs structuraux anioniques répétitifs étant différents des motifs (a), préférablement au moins 10 % en poids, préférablement au moins 20 % en poids des motifs structuraux anioniques répétitifs comprenant une teneur en carbone d'origine biologique de 0 % en poids à 100 % en poids, par rapport à la masse totale de carbone dans le motif structural anionique répétitif, mesurée selon la norme ASTM D6866-12, méthode B.

**2.** Polymère selon la revendication 1, au moins 25 % en poids, ou au moins 30 % en poids, ou au moins 35 % en poids, ou au moins 40 % en poids, ou au moins 45 % en poids, ou au moins 50 % en poids, ou au moins 55 % en poids, ou au moins 60 % en poids, ou au moins 65 % en poids, ou au moins 70 % en poids, ou au moins 75 % en poids, ou au moins 80 % en poids, ou au moins 85 % en poids, ou au moins 90 % en poids, ou au moins 95 % en poids, ou au moins 96 % en poids, ou au moins 97 % en poids, ou au moins 98 % en poids, ou au moins 99 % en poids, ou au moins 99,5 % en poids des motifs répétitifs (a) résultant de l'incorporation d'acryloyldiméthyltaurate comprenant une teneur en carbone d'origine biologique de 28 % en poids à 100 % en poids, par rapport à la masse totale de carbone dans le motif répétitif résultant de l'incorporation d'acryloyldiméthyltaurate, mesurée selon la norme ASTM D6866-12, méthode B.

**3.** Polymère selon l'une quelconque des revendications 1 ou 2, les motifs répétitifs (a) résultant de l'incorporation d'acryloyldiméthyltaurate ayant un degré de neutralisation allant de 50,0 à 100 % en moles, préférablement de 80 % en moles à 100 % en moles, plus préférablement de 90,0 à 100 % en moles, encore plus préférablement de 95,0 à 100 % en moles.

**4.** Polymère selon l'une quelconque des revendications 1 à 3, $Q^+$ étant $H^+$, $Li^+$, $Na^+$, $K^+$, ½ $Ca^{++}$, ½ $Mg^{++}$, ½ $Zn^{++}$, 1/3 $Al^{+++}$, ou des combinaisons correspondantes.

**5.** Polymère selon l'une quelconque des revendications 1 à 3, $Q^+$ étant $Na^+$.

**6.** Polymère selon l'une quelconque des revendications 1 à 5, le polymère comprenant de 37 % en moles à 96,4 % en moles, préférablement de 43 % en moles à 95,3 % en moles de motifs (a), de 0,1 % en moles à 3 % en moles, préférablement de 0,2 % en moles à 2 % en moles de motifs (b), de 0,1 % en moles à 59,3 % en moles, préférablement de 0,5 % en moles à 52,8 % en moles de motifs (c), et de 3,5 % en moles à 16 % en moles, préférablement de 4 % en moles à 14 % en moles de motifs (d).

**7.** Polymère selon l'une quelconque des revendications 1 à 6, l'acryloyldiméthyltaurate comprenant de 35 % en poids, préférablement de 40 % en poids, plus préférablement de 54 % en poids, encore plus préférablement de 57 % en poids à 100 % en poids de teneur en carbone d'origine biologique, par rapport à la masse totale de carbone dans l'acryloyldiméthyltaurate, mesurée selon la norme ASTM D6866-12, méthode B.

**8.** Polymère selon l'une quelconque des revendications 1 à 7, l'acryloyldiméthyltaurate comprenant 100 % en poids de teneur en carbone d'origine biologique, par rapport à la masse totale de carbone dans l'acryloyldiméthyltaurate, mesurée selon la norme ASTM D6866-12, méthode B.

**9.** Polymère selon l'une quelconque des revendications 1 à 8, les motifs de réticulation ou de ramification résultant de l'incorporation d'un monomère selon la formule (4)

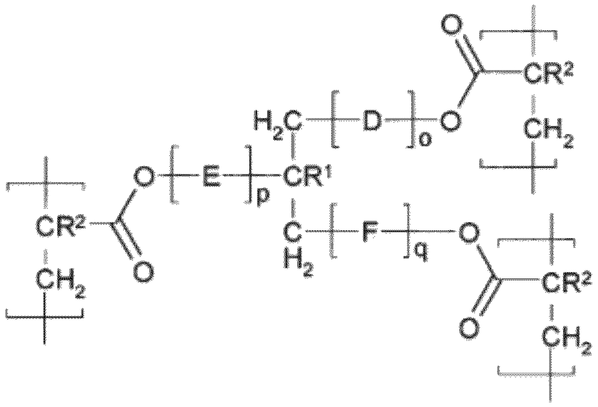

(4)

$R^1$ étant indépendamment choisi parmi H, méthyle ou éthyle ; et
$R^2$ étant un groupe alkyle linéaire ou ramifié ayant 1 à 6 atomes de carbone, ou étant un groupe alkylène linéaire ou ramifié, monoinsaturé ou polyinsaturé ayant 2 à 6 atome de carbone ;
D, E et F étant indépendamment méthylènoxy ($-CH_2O$), éthylènoxy ($-CH_2-CH_2-O-$), propylènoxy ($-CH(CH_3)-CH_2-O-$), un groupe alkylène linéaire ou ramifié ayant 1 à 6 atomes de carbone, un groupe alcénylène linéaire ou ramifié, insaturé une fois ou plusieurs fois, ayant 2 à 6 atomes de carbone, un groupe monohydroxyalkylène linéaire ayant 2 à 6 atomes de carbone ou un groupe dihydroxyalkylène linéaire ou ramifié ayant 3 à 6 atomes de carbone ; et
o, p, et q étant chacun indépendamment un entier de 1 à 50.

**10.** Polymère selon l'une quelconque des revendications 1 à 9, les motifs de réticulation ou de ramification (b) résultant de l'incorporation d'un agent de réticulation choisi dans le groupe constitué par le triacrylate de triméthylolpropane (TMPTA) et/ou le triacrylate de propoxylate de glycérol (GPTA).

**11.** Polymère selon l'une quelconque des revendications 1 à 10, le polymère étant constitué de motifs (a), de motifs (b), de motifs (c) et de motifs (d).

**12.** Polymère selon l'une quelconque des revendications 1 à 11, le polymère ayant un poids moléculaire moyen en poids d'au moins 700 g/mole, préférablement de 700 g/mole à 10 millions g/mole.

**13.** Procédé pour l'obtention d'un polymère par polymérisation de : (a) au moins un monomère d'acryloyldiméthyltaurate selon la formule (10) comprenant une teneur en carbone d'origine biologique de 28 % en poids à 100 % en poids, par rapport à la masse totale de carbone dans le monomère d'acryloyldiméthyltaurate selon la formule (10), mesurée selon la norme ASTM D6866-12, méthode B ; (b) au moins un monomère de réticulation ou de ramification ; (c) au moins un monomère neutre ; et (d) au moins un monomère anionique ;

le monomère de réticulation ou de ramification ayant au moins deux doubles liaisons oléfiniquement insaturées ; et la formule (10) étant :

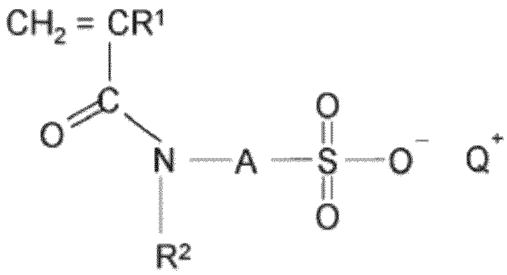

(10)

$R^1$ et $R^2$ étant H ; A étant $-C(CH_3)_2-H_2C-$ ; et
le monomère d'acryloyldiméthyltaurate étant préparé à partir d'acrylonitrile, d'isobutène et d'un mélange d'acide sulfurique et d'acide sulfurique fumant comprenant du trioxyde de soufre, au moins l'un parmi l'acrylonitrile et l'isobutène étant d'origine biologique, et

$Q^+$ étant $H^+$, $NH_4^+$, des ions ammonium organiques $[NHR^5R^6R^7]^+$, $R^5$, $R^6$, et $R^7$ indépendamment les uns des autres pouvant être hydrogène, un groupe alkyle linéaire ou ramifié ayant 1 à 22 atomes de carbone, un groupe alcényle linéaire ou ramifié, insaturé une fois ou plusieurs fois, ayant 2 à 22 atomes de carbone, un groupe alkylamidopropyle en $C_{6-22}$, un groupe monohydroxyalkyle linéaire ayant 2 à atomes de carbone ou un groupe dihydroxyalkyle linéaire ou ramifié ayant 3 à atomes de carbone, et au moins l'un des radicaux $R^5$, $R^6$, et $R^7$ n'étant pas hydrogène, ou $Q^+$ étant $Li^+$, $Na^+$, $K^+$, ½ $Ca^{++}$, ½ $Mg^{++}$, ½ $Zn^{++}$, 1/3 $Al^{+++}$, ou des combinaisons correspondantes.

**14.** Utilisation du polymère selon l'une quelconque des revendications 1 à 12 en tant qu'agent épaississant et/ou agent de modification de la rhéologie et/ou agent de modification de la viscosité.

**15.** Composition comprenant :

(a) un polymère selon l'une quelconque des revendications 1 à 12 ; et
(b) un composant supplémentaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0816403 A
- WO 9800094 A
- US 20140086854 A
- US 20140154758 A
- DE 2655891
- US 4138430 A
- US 20100048850 A1
- WO 2009063120 A1
- US 2904580 A
- WO 2014086780 A
- WO 2016042011 A
- WO 2014004616 A
- WO 2015034948 A
- WO 2013113938 A
- WO 2014086780 A2
- EP 16175218


### Non-patent literature cited in the description


- **DE JONG et al.** Product developments in the bio-based chemicals arena. *Biofuels, Bioprod. Bioref.,* 2012, vol. 6, 606-624
- **M. OLGA GUERRERO-PÉREZA ; MIGUEL A. BAÑARES.** *Catalysis Today,* 2015, vol. 239, 25-30
- **M.O. GUERRERO-PÉREZ ; M.A. BAÑARES.** *ChemSusChem,* 2008, vol. 1, 511
- **M.A. BAÑARES ; M.O. GUERRERO-PÉREZ.** *Appl. Catal. B,* 2013
- **GEORG ODIAN.** Principles of Polymerization. Wiley-Interscience, 19-24
- **BERND TIEKE.** Makromolekulare Chemie: Eine Einführung. Wiley-VCH
- *CHEMICAL ABSTRACTS,* 8014-95-7
- **MASSAO KUNIOKA.** *Radioisotopes,* 2013, vol. 62, 901-925
- **BIANCA et al.** *Appl Microbiol Biotechnol,* 2012, vol. 93, 1377-1387